# EUROPÄISCHE PATENTSCHRIFT

(11) **EP 1 685 096 B1**
(45) Veröffentlichungstag und Bekanntmachung des Hinweises auf die Patenterteilung: **25.07.2007**
(21) Anmeldenummer: 04797535.4
(22) Anmeldetag: 03.11.2004
(51) Int. Cl.: C07C 381/00, A61K 31/166, A61P 9/06, A61P 9/10

(54) **PENTAFLUORSULFANYL-BENZOYLGUANIDINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
PENTAFLUOROSULFANYL BENZOYLGUANIDINES, METHOD FOR THEIR PRODUCTION, THEIR USE AS MEDICAMENTS OR DIAGNOSTIC AGENTS AND MEDICAMENT CONTAINING THE SAME
PENTAFLUORSULFANYL-BENZOYLGUANIDINES, PROCEDE POUR LES PREPARER, LEUR UTILISATION COMME MEDICAMENT OU COMME AGENT DE DIAGNOSTIC ET MEDICAMENT LES CONTENANT

(30) Priorität: 13.11.2003 DE 10353202
(43) Veröffentlichungstag der Anmeldung: 02.08.2006
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE)
(86) Internationale Anmeldenummer: PCT/EP2004/012393
(87) Internationale Veröffentlichungsnummer: WO 2005/047239

(56) Entgegenhaltungen:
- EP-A- 0 589 336
- EP-A- 0 686 627
- EP-A- 0 743 301
- EP-A- 0 754 680

## Beschreibung

### Pentafluorsulfanyl-benzoylguanidine der Formel I

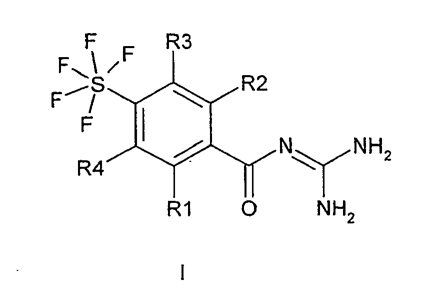

worin R1 bis R4 die in den Ansprüchen angegebenen Bedeutungen haben, und deren pharmazeutisch verträgliche Salze sind substituierte Acylguanidine und inhibieren den zellulären Natrium-Protonen-Antiporter (Na⁺/H⁺-Exchanger, NHE). Aufgrund der NHE-inhibitorischen Eigenschaften eignen sich die Verbindungen der Formel I und deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

Gegenüber den bekannten Verbindungen zeichnen sich die erfindungsgemäßen Verbindungen durch eine außerordentlich hohe Wirksamkeit in der Inhibition des Na⁺/H⁺-Austauschs, sowie durch verbesserte ADMET-Eigenschaften aus. Durch die xenobiotische Struktur (insbesondere durch die Einführung der eher "unnatürlichen/naturfremden" SF₅-Substituenten) wird die Gewebeverteilung vorteilhaft beeinflusst. Das führt u.a. zu erhöhten Expositionen in vivo. Dabei wird das Resorptionsverhalten nicht signifikant beeinflusst und die hohe Bioverfügbarkeit der Acylguanidine bleibt erhalten.

Im Gegensatz zu einigen in der Literatur (zum Beispiel in EP 0 686 627) beschriebenen Acylguanidinen zeigen die hier beschriebenen Verbindungen der Formel I und deren pharmazeutisch verträgliche Salze keine unerwünschten und nachteiligen saliduretischen Eigenschaften.

Die Erfindung betrifft Pentafluorsulfanyl-benzoylguanidine der Formel I 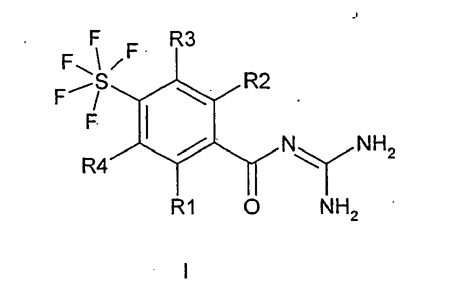 worin bedeuten
- R1: Wasserstoff, Alkyl mit 1, 2, 3 oder 4 C-Atomen, Alkoxy mit 1, 2, 3 oder 4 C Atomen, F, Cl, Br, I, -CN, NR5R6, -Oₚ-(CH₂)ₙ-(CF₂)ₒ-CF₃ oder -(SOₘ)_{q}-(CH₂)ᵣ-(CF₂)ₛ-CF₃;
R5 und R6 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
- R2: Wasserstoff, Alkyl mit 1, 2, 3 oder 4 C-Atomen, Alkoxy mit 1, 2, 3 oder 4 C-Atomen, F, Cl, Br, I, -CN, NR7R8, -Oₜ-(CH₂)ᵤ-(CF₂)ᵥ-CF₃ oder -(SO_{w})ₓ-(CH₂)_{y}-(CF₂)_{z}-CF₃;
R7 und R8 unabhängig voneinander Wasserstoff, Alkyl mit 1, 2, 3 oder 4 C-Atomen oder -CH₂-CF₃;
w Null, 1 oder 2
t, u, v, x, y und z unabhängig voneinander Null oder 1;
- R3: Cl, Br, I, -CN, -SO₂CH₃, Alkoxy mit 1, 2, 3 oder 4 C-Atomen, NR9R10, -Oₐ-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
a, b und c unabhängig voneinander Null oder 1;
d Null, 1 oder 2;
e Null oder 1;
f Null, 1, 2, 3 oder 4;
g Null oder 1;
oder
- R3: -(CH₂)ₕ-Phenyl oder -O-Phenyl,
in denen die Phenylreste unsubstituiert sind oder substituiert sind mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃ ;
j Null oder 1;
k Null, 1, 2 oder 3;
h Null, 1, 2, 3 oder 4;
oder
- R3: -(CH₂)ₐₐ -Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
aa Null, 1, 2, 3 oder 4;
- R4: Wasserstoff, F, Cl, Br, I, -CN, -SO₂CH₃, Alkoxy mit 1, 2, 3 oder 4 C-Atomen, NR11R12, -O_{dd}-(CH₂)ₑₑ-(CF₂)_{ff}-CF₃, -(SO_{gg})ₕₕ-(CH₂)ⱼⱼ-(CF₂)ₖₖ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R11 und R12 unabhängig voneinander Wasserstoff, Alkyl mit 1, 2, 3 oder 4 C-Atomen oder -CH₂-CF₃;
dd, ee und ff unabhängig voneinander Null oder 1;
gg Null, 1 oder 2;
hh Null oder 1;
jj Null, 1, 2, 3 oder 4;
kk Null oder 1;
oder
- R4: -(CH₂)ₗₗ -Phenyl oder -O-Phenyl,
in denen die Phenylreste unsubstituiert sind oder substituiert sind mit 1, 2 oder 3 Resten ausgewählt aus der Gruppe bestehend aus F, Cl, Br, I, -Oₘₘ-(CH₂)ₙₙ-CF₃, Alkoxy mit 1, 2, 3 oder 4 C-Atomen, Alkyl mit 1, 2, 3 oder 4 C-Atomen und -SO₂CH₃;
mm Null oder 1;
nn Null, 1, 2 oder 3;
ll Null, 1, 2, 3 oder 4;
oder
- R4: -(CH₂)ₒₒ -Heteroaryl,
das unsubstituiert ist oder substituiert ist mit 1, 2 oder 3 Resten ausgewählt aus der Gruppe bestehend aus F, Cl, Br, I, -Oₚₚ-(CH₂)ᵣᵣCF₃, Alkoxy mit 1, 2, 3 oder 4 C-Atomen, Alkyl mit 1, 2, 3 oder 4 C-Atomen und -SO₂CH₃ ;
pp Null oder 1 ;
rr Null, 1, 2 oder 3;
oo Null, 1, 2, 3 oder 4;
sowie deren pharmazeutisch verträgliche Salze.

Bevorzugt sind Verbindungen der Formel I, in denen bedeuten:
- R1: Wasserstoff, Alkyl mit 1, 2, 3 oder 4 C-Atomen, Alkoxy mit 1, 2, 3 oder 4 C-Atomen, F, Cl, Br, I, -CN, NR5R6, -Oₚ-(CH₂)ₙ-(CF₂)ₒ-CF₃ oder -(SOₘ)_{q}-(CH₂)ᵣ-(CF₂)ₛ-CF₃;
R5 und R6 unabhängig voneinander Wasserstoff, Alkyl mit 1, 2, 3 oder 4 C-Atomen oder -CH₂-CF₃;
m Null, 1 oder 2
n, o, p, q, rund s unabhängig voneinander Null oder 1;
- R2: Wasserstoff oder F;
- R3: Cl, Br, I, -CN, -SO₂CH₃ Alkoxy mit 1, 2, 3 oder 4 C-Atomen, NR9R10, -Oₐ-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
a, b und c unabhängig voneinander Null oder 1;
d Null, 1 oder 2;
e Null oder 1;
f Null, 1, 2, 3 oder 4;
g Null oder 1;
oder
- R3: -(CH₂)ₕ -Phenyl oder -O-Phenyl,
in denen die Phenylreste unsubstituiert sind oder substituiert sind mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
h Null, 1, 2, 3 oder 4;
oder
- R3: -(CH₂)ₐₐ -Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
aa Null, 1, 2, 3 oder 4;
- R4: Wasserstoff oder F;
sowie deren pharmazeutisch verträgliche Salze.

Besonders bevorzugt sind Verbindungen der Formel I, in denen bedeuten:
- R1: Wasserstoff, Alkyl mit 1, 2, 3 oder 4 C-Atomen, Alkoxy mit 1, 2, 3 oder 4 C-Atomen, F, Cl, Br, I, -CN, NR5R6, -O-CH₂-CF₃ oder -(SOₘ)_{q}-(CH₂)ᵣCF₃;
R5 und R6 unabhängig voneinander Wasserstoff, Alkyl mit 1, 2, 3 oder 4 C-Atomen oder -CH₂-CF₃;
m Null, 1 oder 2
q und r unabhängig voneinander Null oder 1;
- R2: Wasserstoff oder F;
- R3: Cl, Br, I, -CN, -SO₂CH₃, Alkoxy mit 1, 2, 3 oder 4 C-Atomen, NR9R10, . -O-CH₂-CF₃, -(SO_{d})ₑ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
d Null, 1 oder 2 ;
e Null oder 1;
oder
- R3: Phenyl,
das unsubstituiert ist oder substituiert ist mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
- j: Null oder 1;
- k: Null, 1, 2 oder 3;
oder
- R3: Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
- R4: Wasserstoff oder F;
sowie deren pharmazeutisch verträgliche Salze.

Ganz besonders bevorzugt sind Verbindungen der Formel I, in denen bedeuten:
- R1: Wasserstoff, Alkyl mit 1, 2, 3 oder 4 C-Atomen, Methoxy, Ethoxy, F, Cl, NR5R6, -O-CH₂-CF₃ oder -(SOₘ)_{q}-(CH₂)ᵣ-CF₃;
R5 und R6 unabhängig voneinander Wasserstoff, Alkyl mit 1, 2, 3 oder 4 C-Atomen oder -CH₂-CF₃;
m Null, 1 oder 2
q und r unabhängig voneinander Null oder 1;
- R2: Wasserstoff oder F;
- R3: Cl, -CN, -SO₂CH₃, Methoxy, Ethoxy, NR9R10, -O-CH₂-CF₃, -(SO_{d})ₑ-CF₃, Alkyl mit 1, 2, 3, 4, 5 oder 6 C-Atomen oder Cycloalkyl mit 3, 4, 5, 6 oder 7 C-Atomen, in dem 1, 2, 3 oder 4 Wasserstoff-Atome durch Fluoratome ersetzt sein können;
R9 und R10 unabhängig voneinander Wasserstoff, Methyl, Ethyl oder -CH₂-CF₃;
d Null, 1 oder 2;
e Null oder 1;
oder
- R3: Phenyl,
das unsubstituiert ist oder substituiert ist mit 1 oder 2 Resten ausgewählt aus der Gruppe bestehend aus F, Cl, -Oⱼ-(CH₂)ₖ-CF₃, Methoxy, Ethoxy, Alkyl mit 1, 2, 3 oder 4 C-Atomen und -SO₂CH₃;
j und k unabhängig voneinander Null oder 1;
oder
- R3: Heteroaryl,
das unsubstituiert ist oder substituiert ist mit 1 oder 2 Resten ausgewählt aus der Gruppe bestehend aus F, Cl, -O_{bb}-(CH₂)_{cc}-CF₃, Methoxy, Ethoxy, Alkyl mit 1, 2, 3 oder 4 C-Atomen und -SO₂CH₃;
bb und cc unabhängig voneinander Null oder 1;
- R4: Wasserstoff oder F;
sowie deren pharmazeutisch verträgliche Salze.

In einer Ausführungsform sind dabei Verbindungen der Formel I bevorzugt, in denen R1 durch Wasserstoff, Alkyl mit 1, 2, 3 oder 4 C-Atomen, Alkoxy mit 1, 2, 3 oder 4 C-Atomen, F, Cl, Br, I, -CN, NR5R6, wobei R5 und R6 unabhängig voneinander Wasserstoff, Alkyl mit 1, 2, 3, oder 4 C-Atomen oder -CH₂-CF₃ sind, -O-CH₂-CF₃ oder -(SOₘ)_{q}-(CH₂)ᵣ-CF₃, wobei m Null, 1 oder 2 ist und q und r unabhängig voneinander Null oder 1 sind, beschrieben wird; besonders bevorzugt sind Verbindungen, in denen R1 durch Wasserstoff, Alkyl mit 1, 2, 3 oder 4 C-Atomen, Methoxy, Ethoxy, F, Cl, NR5R6, wobei R5 und R6 unabhängig voneinander Wasserstoff, Alkyl mit 1, 2, 3, oder 4 C-Atomen oder -CH₂-CF₃ sind, -O-CH₂-CF₃ oder -(SOₘ)_{q}-(CH₂)ᵣ-CF₃, wobei m Null, 1 oder 2 ist und q und r unabhängig voneinander Null oder 1 sind, beschrieben wird; ganz besonders bevorzugt sind Verbindungen, in denen R1 durch Wasserstoff, Methyl, Ethyl, CF₃-CH₂-O-, F, Cl oder CF₃ beschrieben wird. In einer weiteren Ausführungsform sind Verbindungen bevorzugt, in denen R1 durch Wasserstoff, Methyl oder Ethyl, insbesondere Methyl oder Ethyl, beschrieben wird.

In einer weiteren Ausführungsform sind Verbindungen der Formel I bevorzugt, in denen R2 durch Wasserstoff oder F beschrieben wird; besonders bevorzugt sind Verbindungen, in denen R2 durch Wasserstoff beschrieben wird.

In einer weiteren Ausführungsform sind Verbindungen der Formel I bevorzugt, in denen R3 durch Cl, -CN, -SO₂CH₃, Methoxy, Ethoxy, NR9R10, wobei R9 und R10 unabhängig voneinander Wasserstoff, Methyl, Ethyl oder -CH₂-CF₃ sind, -O-CH₂-CF₃, -(SO_{d})ₑ-CF₃, wobei d Null, 1 oder 2 ist und e Null oder 1 ist, Alkyl mit 1, 2, 3, 4, 5 oder 6 C-Atomen oder Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit 1, 2 oder 3 Resten ausgewählt aus der Gruppe bestehend aus F, Cl, Br, I, -Oⱼ-(CH₂)ₖ-CF₃, wobei j Null oder 1 ist und k Null, 1, 2 oder 3 ist, Alkoxy mit 1, 2, 3 oder 4 C-Atomen, Alkyl mit 1, 2, 3 oder 4 C-Atomen und -SO₂CH₃, oder Heteroaryl, das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, wobei bb Null oder 1 ist und cc Null, 1, 2 oder 3 ist, Alkoxy mit 1, 2, 3 oder 4 C-Atomen, Alkyl mit 1, 2, 3 oder 4 C-Atomen und -SO₂CH₃, beschrieben wird; besonders bevorzugt sind Verbindungen, in denen R3 durch Cl, -CN, -SO₂CH₃, Methoxy, Ethoxy, NR9R10, wobei R9 und R10 unabhängig voneinander Wasserstoff, Methyl, Ethyl oder -CH₂-CF₃ sind, -O-CH₂-CF₃, -(SO_{d})ₑ-CF₃, wobei d Null, 1 oder 2 ist und e Null oder 1 ist, Alkyl mit 1, 2, 3, 4, 5 oder 6 C-Atomen oder Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit 1 - 2 Resten ausgewählt aus der Gruppe bestehend aus F, Cl, -Oⱼ-(CH₂)ₖ-CF₃, wobei j und k unabhängig voneinander Null oder 1 sind, Methoxy, Ethoxy, Alkyl mit 1, 2, 3 oder 4 C-Atomen und -SO₂CH₃, oder Heteroaryl, das unsubstituiert ist oder substituiert ist mit 1 - 2 Resten ausgewählt aus der Gruppe bestehend aus F, Cl, -O_{bb}-(CH₂)_{cc}-CF₃, wobei bb und cc unabhängig voneinander Null oder 1 sind, Methoxy, Ethoxy, Alkyl mit 1, 2, 3 oder 4 C-Atomen und -SO₂CH₃, beschrieben wird; ganz besonders bevorzugt sind Verbindungen, in denen R3 durch Cl, -CN oder -SO₂CH₃ beschrieben wird.

In einer weiteren Ausführungsform sind Verbindungen der Formeln I bevorzugt, in denen R4 durch Wasserstoff und F beschrieben wird, besonders bevorzugt sind Verbindungen, in denen R4 durch Wasserstoff beschrieben wird.

In einer weiteren Ausführungsform sind Verbindungen der Formel I bevorzugt, in denen p, t, a und dd unabhängig voneinander 1 sind.

Enthalten die Substituenten R1 bis R4 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl. Substituierte Alkylreste können in beliebigen Positionen substituiert sein.
Beispiele für Cycloalkylreste sind Cyclopropyl, Cyclobutyl, Cyclopentyl, Cyclohexyl Cycloheptyl oder Cyclooctyl.

Substituierte Cycloalkylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie zum Beispiel Phenylalkyl oder Phenyloxy. In monosubstituierten Phenylresten kann sich der Substituent in der 2-Position, der 3-Position öder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über die 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4-oder -5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder -5-yl, 1,2,4-Oxadiazol-3- oder -5-yl, 1,3,4-Oxadiazol-2-yl oder-5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, -3- oder - 4-pyridyl.

Besonders bevorzugt sind die Heteroaromaten 2- oder 3-Thienyl, 2- oder 3-Furyl, 1-, 2- oder 3- Pyrrolyl, 1-, 2-, 4- oder 5-Imidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-; 4-, 5- oder 6-Pyrimidinyl und 3- oder 4-Pyridazinyl.

Die Erfindung betrifft weiterhin ein Verfahren zur Herstellung der Verbindungen der Formel I, dadurch gekennzeichnet, dass man eine Verbindung der Formel II 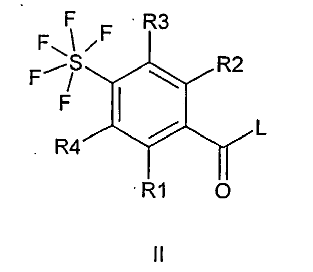 worin R1 bis R4 die angegebene Bedeutung besitzen und L für eine nucleophil substituierbare Fluchtgruppe steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in dem Fachmann bekannter Weise aus den zugrundeliegenden Carbonsäurechloriden (Formel II; L = Cl), die man ihrerseits wiederum in bekannter Weise aus den zugrundeliegenden Carbonsäuren (Formel II; L = OH) beispielsweise mit Thionylchlorid herstellen kann.
Neben den Carbonsäurechloriden der Formel II (L = Cl) lassen sich auch weitere aktivierte Säurederivate der Formel II in bekannter Weise direkt aus den zugrundeliegenden Benzoesäuren (Formel II; L = OH) herstellen, wie die Methylester der Formel II mit L = OCH₃ durch Behandeln mit gasförmigem HCl in Methanol, die Imidazolide der Formel II durch Behandeln mit Carbonyldiimidazol, die gemischten Anhydride der Formel ll durch Behandeln mit Cl-COOC₂H₅ oder Tosylchlorid in Gegenwart von Triethylamin in einem inerten Lösungsmittel, wie auch Aktivierungen von Benzoesäuren mit Dicyclohexylcarbodümid (DCC) oder mit O-[(Cyano(ethoxycarbonyl)methylen)amino]-1,1,3,3-tetramethyluronium-tetrafluorborat ("TOTU") möglich sind. Eine Reihe geeigneter Methoden zur Herstellung von aktivierten Carbonsäurederivaten der Formel ll sind unter Angabe von Quellenliteratur in J. March, Advanced Organic Chemistry, Third Edition (John Wiley & Sons, 1985, S. 350) angegeben.
Die Umsetzung eines aktivierten Carbonsäurederivates der Formel II mit Guanidin erfolgt vorzugsweise in bekannter Weise in einem protischen oder aprotischen polaren aber inerten organischen Lösungsmittel. Dabei haben sich bei der Umsetzung der Benzoesäuremethytester (Formel ll; L = OCH₃) mit Guanidin Methanol, lsopropanol oder THF bei Temperaturen von 20°C bis zur Siedetemperatur dieser Lösungsmittel. bewährt. Bei den meisten Umsetzungen von Verbindungen der Formel ll mit salzfreiem Guanidin wird beispielsweise in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von Verbindungen der Formel II mit Guanidin verwendet werden.

Wenn L die Bedeutung Cl hat, arbeitet man vorteilhaft unter Zusatz eines Säurefängers, zum Beispiel in Form von überschüssigem Guanidin, zur Abbindung der Halogenwasserstoffsäure.

Die Verbindungen der Formel II können wie folgt hergestellt werden, indem 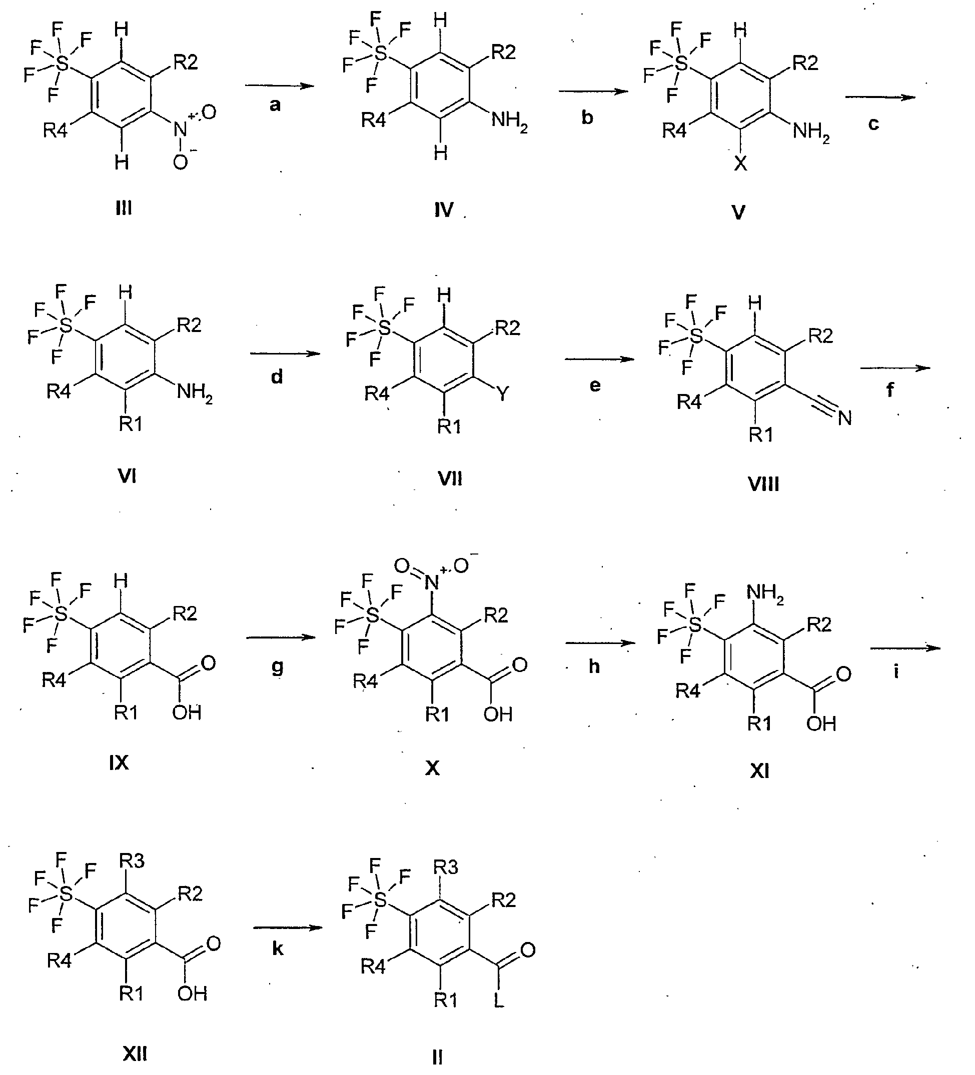
a) ein 4-Nitrophenyl-schwefelpentafluorid-Derivat der Formel III zum Amin der Formel IV reduziert wird,
b) die Verbindung der Formel IV in ortho-Stellung zur Aminogruppe mit einem Halogenierungsmittel zur Verbindung der Formel V halogeniert wird,
c) in der Verbindung der Formel V mit einem geeigneten Nukleophil oder einer Organoelementverbindung, beispielsweise einer Alkylborverbindung, gegebenenfalls unter Katalyse, der Halogen-Substituent durch einen Substituenten R1 ersetzt wird, und
d) in der Verbindung der Formel VI die Aminofunktion durch einen HalogenSubstituenten ersetzt wird,
e) in der Verbindung der Formel VII der Halogen-Substituent durch eine Nitrilfunktion ersetzt wird,
f) die Nitrilfunktion der Verbindung der Formel VIII zur Carbonsäure hydrolysiert wird,
g) die Verbindung der Formel IX in ortho-Stellung zur Pentafluorsulfanylgruppe zur Verbindung der Formel X nitriert wird,
h) die Nitroverbindung der Formel X zum Anilin reduziert wird,
i) in der Verbindung der Formel XI die Aminofunktion mit einem geeigneten Nucleophil durch R3 ersetzt wird
   und
k) die Verbindung der Formel XII in die Verbindung der Formel II umgewandelt wird, wobei in den Verbindungen der Formeln II, III, IV, V, VI, VII, VIII, IX, X, XI und XII II
R1 bis R4 definiert sind wie in Formel I
L definiert ist wie in Formel II und
X und Y unabhängig voneinander F, Cl, Br oder I sind.

Bei der Herstellung der Verbindungen der Formel II geht man beispielsweise so vor, dass zunächst in Schritt a die Verbindungen der Formel III nach bekannten Methoden zur Reduktion von aromatischen Nitroverbindungen zu aromatischen Aminen zu Verbindungen der Formel IV umgesetzt werden. Solche Methoden sind beispielsweise beschrieben in: R.C. Larock, Comprehensive Organic Transformations: A Guide to Functional Group Preparations, VCH Publishers, New York, Weinheim, 1999, 821-828 und der dort zitierten Literatur.

Anschließend (Schritt b) werden die Verbindungen der Formel IV in einem organischen Lösungsmittel A gelöst und mit einem Halogenierungsmittel, beispielsweise mit einem Bromierungsmittel, umgesetzt. Die Reaktionstemperatur beträgt dabei im allgemeinen -30 °C bis +150°C, bevorzugt 0 °C bis 40 °C. Die Reaktionszeit liegt im allgemeinen bei 10 min bis 20 h, je nach Zusammensetzung des Gemisches und des gewählten Temperaturbereiches. Zur Aufarbeitung kann das erhaltene Reaktionsgemisch anschließend über eine Kieselgelschicht filtriert, mit organischem Lösungsmittel A nachgewaschen und nach dem Entfernen des Lösungsmittels im Vakuum das Produkt durch übliche Reinigungsmethoden wie Umkristallisieren, Destillation oder Chromatographie gereinigt werden.
Es werden beispielsweise 0,1 bis 10 Mol der Verbindung der Formel IV in 1000 mL organischem Lösungsmittel A gelöst. Beispielsweise verwendet man auf 1 Mol der zu halogenierenden Verbindung der Formel IV 0,8 bis 1,2 Äquivalente des Halogenierungsmittels.
Unter dem Begriff "Halogenierungsmittel" werden zum Beispiel elementare Halogene, Halogen-Amin-Komplexe, cyclische und nichtcyclische N-halogenierte Carbonsäure-amide und -imide sowie Harnstoffe verstanden, wie sie beispielsweise in R.C. Larock, Comprehensive Organic Transformations: A Guide to Functional Group Preparations, VCH Publishers, New York, Weinheim, 1999, 619-628 und der dort zitierten Literatur oder M.B. Smith and J. March, March's Advanced Organic Chemistry: Reactions, Mechanisms, and Structure, Wiley, New York, 2001, 704-707 und der dort zitierten Literatur beschrieben sind, wie zum Beispiel N-Bromsuccinimid, N-Chlorsuccinimid, HBr in H₂SO₄ oder 1,3-Dibrom-5,5-dimethyl-imidazolidin-2,4-dion. Unter dem Begriff "Bromierungsmittel" werden zum Beispiel elementares Brom, Brom-Amin-Komplexe, cyclische und nichtcyclische N-bromierte Carbonsäure-amide und -imide sowie Harnstoffe verstanden, wie sie beispielsweise in R.C. Larock, Comprehensive Organic Transformations: A Guide to Functional Group Preparations, VCH Publishers, New York, Weinheim, 1999, 622-624 und der dort zitierten Literatur oder M.B. Smith and J. March, March's Advanced Organic Chemistry: Reactions, Mechanisms, and Structure, Wiley, New York, 2001, 704-707 und der dort zitierten Literatur beschrieben sind, beispielsweise N-Bromsuccinimid, HBr in H₂SO₄ oder 1,3-Dibrom-5,5-dimethylimidazolidin-2,4-dion, wobei letzteres 2 Bromatome pro Molekül übertragen kann.

Unter dem Begriff "organisches Lösungsmittel A" werden vorzugsweise aprotische Lösungsmittel wie beispielsweise Dichlormethan, Chloroform, Tetrachlormethan, Pentan, Hexan, Heptan, Oktan, Benzol, Toluol, Xylol, Chlorbenzol, 1,2-Dichlorethan, Trichlorethylen oder Acetonitril verstanden.
Eventuell bei der Reaktion entstehendes HX kann durch organische oder anorganische Basen abgefangen werden.

In Schritt c werden die Verbindungen der Formel V anschließend in einem organischen Lösungsmittel B gelöst und mit einen Nukleophil R1⁻ oder einer Elementverbindung, die den Substituenten R1 enthält, zu Verbindungen der Formel VI umgesetzt. Dabei kann unter Zusatz einer Base A gearbeitet werden und ein katalysierendes Metallsalz A zugegeben werden.
Die Reaktionstemperatur beträgt dabei im allgemeinen zwischen -20°C und +150 °C, bevorzugt zwischen 30 °C und 100 °C. Die Reaktionszeit liegt im allgemeinen bei 0,5 h bis 20 h, je nach Zusammensetzung des Gemisches und des gewählten Temperaturbereiches. Zur Aufarbeitung kann das erhaltene Reaktionsgemisch anschließend über eine Kieselgelschicht abfiltriert, mit einem organischen Lösungsmittel B nachgewaschen und nach dem Entfernen des Lösungsmittels im Vakuum das Produkt durch übliche Reinigungsverfahren wie Umkristallisieren, Chromatographie, zum Beispiel an Kieselgel, Destillation oder Wasserdampfdestillation gereinigt werden.
Es werden beispielsweise 0,1 bis 10 Mol der Verbindung der Formel V in 1000 mL organischem Lösungsmittel B gelöst. Beispielsweise verwendet man auf 1 Mol der Ausgangsverbindung der Formel V 0,8 bis 3 Äquivalente des Nucleophils R1- oder der Elementverbindung, die den Substituenten R1 enthält.
Unter dem Begriff "Nucleophil R1⁻"werden Verbindungen verstanden, die beim Deprotonieren einer Verbindung R1-H mit starken Basen, wie beispielsweise Alkyl- oder Aryllithiumverbindungen, Organomagnesiumverbindungen, Alkoholaten oder Lithiumdiisopropylamid, entstehen.
Unter "Organoelementverbindungen, die den Substituenten R1 enthalten" werden beispielsweise Organolithiumverbindungen R1-Li, Organomagnesiumverbindungen R1-Mg-Hal, mit Hal = Cl, Br, I, Organoborverbindungen wie R1-B(OH)₂, R1-Boronsäureester wie beispielsweise 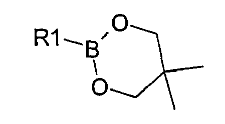 R2-Boronsäureanhydride wie beispielsweise 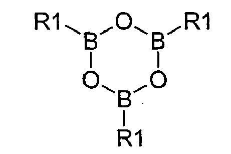 oder Organozinkverbindungen R1-Zn-Z, mit Z = Cl, Br, I, verstanden.
Unter dem Begriff "Base A" werden Basen verstanden, wie sie als Hilfsbasen bei Kreuzkupplungsreaktionen verwendet werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263-303 und der darin jeweils zitierten Literatur aufgeführt sind, beispielsweise Na₂CO₃, C_{S2}CO₃, KOH, NaOH, K₃PO₄, N(Ethyl)₃.
Unter dem Begriff "organisches Lösungsmitel B" werden protische oder aprotische Lösungsmittel verstanden wie Diethylether, Dimethoxyethan, THF, Alkohole, Wasser oder deren Gemische. In einer Ausführungsform sind Gemische mit Wasser bevorzugt.
Unter dem Begriff "katalysierendes Metallsalz A" werden unter anderem Pd- und Ni-Katalysatoren verstanden, wie sie für Suzuki- und Negishi-Reaktionen verwendet werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263 oder G.C. Fu et al., J. Am. Chem. Soc. 2001, 123, 10099 oder G.C. Fu et al., J. Am. Chem. Soc. 2002, 124, 13662 und der dort jeweils zitierten Literatur beschrieben sind, einschließlich der zugesetzten Liganden, wie Pd(OAc)₂, PdCl₂(dppf) oder Pd₂(dba)₃.

In Schritt d werden anschließend die Verbindungen der Formel VI durch ein Diazotierungs-Halogenierungsverfahren mit einem Diazotierungs-Halogenierungsmittel, beispielsweise mit einem Diazotierungs-Bromierungsmittel, wie es für andere aromatische Amine zum Austausch der Amin- gegen eine Halogen-Funktion beispielsweise in M.B. Smith and J. March, March's Advanced Organic Chemistry: Reactions, Mechanisms, and Structure, Wiley, New York, 2001, 935-936 oder R.C. Larock, Comprehensive Organic Transformations: A Guide to Functional Group Preparations, VCH Publishers, New York, Weinheim, 1999, 678-679 und der dort zitierten Literatur beschrieben wurde, beispielsweise durch die Sandmeyer- oder Gattermann-Reaktion, in die Verbindungen der Formel Vll überführt. Bevorzugt ist das Verfahren von M. Doyle et al., J. Org. Chem. 1977, 42, 2426 oder von S. Oae et al., Bull. Chem. Soc. Jpn. 1980, 53, 1065.

In Schritt e werden die Verbindungen der Formel VII in einem Löungsmittel C mit einem Cyanierungsmittel, zum Beispiel unter Zusatz eines katalysierenden Metallsalzes B, umgesetzt. Die Reaktionstemperatur beträgt im allgemeinen 20 °C bis 200 °C, bevorzugt 80 °C bis 150 °C. Die Reaktionszeit liegt im allgemeinen bei 1 h bis 20 h, je nach Zusammensetzung des Gemisches und des gewählten Temperaturbereiches. Die erhaltenen Reaktionsgemische können über eine Kieselgel- oder Kieselgurschicht abgesaugt und das Filtrat wässrig-extraktiv aufgearbeitet. Nach dem Eindampfen des Lösungsmittels im Vakuum wird die Verbindung der Formel VIII durch übliche Reinigungsverfahren wie Umkristallisieren, Chromatographie an Kieselgel, Destillation oder Wasserdampfdestillation gereinigt.
Es werden beispielsweise 0,1 bis 10 Mol der Verbindung der Formel VII in 1000 mL organischem Lösungsmittel C gelöst. Beispielsweise verwendet man auf 1 Mol der umzusetzenden Verbindungen mit der Formel VII 1 bis 10 Äquivalente des Cyanierungsmittels.
Unter dem Begriff "Cyanierungsmittel" werden beispielsweise Alkalimetallcyanide oder Zn(CN)₂ entweder alleine oder im Gemisch mit metallischem Zink, bevorzugt in Form von Zink-Staub, verstanden.
Unter dem Begriff "organisches Lösungsmittel C" werden vorzugsweise aprotische polare Lösungsmittel wie beispielsweise DMF, Dimethylacetamid, NMP, DMSO verstanden.

Unter dem Begriff "katalysierendes Metallsalz B" werden unter anderem Pd- und Ni-Katalysatoren verstanden, wie sie bei Suzuki-Reaktionen eingesetzt werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263 und der dort zitieten Literatur beschrieben werden, beispielsweise PdCl₂(dppf), Pd(OAc)₂, Pd₂(dba)₃.

Anschließend werden die erhaltenen Verbindungen der Formel VIII in Schritt f, zum Beispiel in Gegenwart einer Base, zu den Carbonsäuren der Formel IX hydrolysiert. Dies kann geschehen durch dem Fachmann bekannte Verfahren zur Hydrolyse aromatischer Nitrile, wie sie beispielsweise in R.C. Larock, Comprehensive Organic Transformations: A Guide to Functional Group Preparations, VCH Publishers, New York, Weinheim, 1999, 1986-1987 oder M.B. Smith and J. March, March's Advanced Organic Chemistry: Reactions, Mechanisms, and Structure, Wiley, New York, 2001, 1179-1180 und der dort zitierten Literatur beschrieben sind.

In Schritt g werden Verbindungen der Formel IX mit einem Nitrierungsmittel, wie beispielsweise in Houben-Weyl, Methoden der organischen Chemie 4. Auflage, Organo-Stickstoff-Verbindungen IV, Teil 1, Georg Thieme Verlag Stuttgart 1992, S. 262-341 beschrieben nitriert.

In Schritt h werden die Nitroverbindungen der Formel X nach im Prinzip bekannten Methoden zur Reduktion von aromatischen Nitroverbindungen zu aromatischen Aminen zu Verbindungen der Formel XI umgesetzt. Solche Methoden sind beispielsweise beschrieben in: R.C. Larock, Comprehensive Organic Transformations: a Guide to Functional Group Preparations, VCH Publishers, New York, Weinheim, 1999, 821-828 und der dort zitierten Literatur.

In Schritt i werden die Aniline der Formel XI über den Weg der Diazotierung-Substitution in die Verbindungen der Formel XII überführt unter Substitution der Amingruppe durch R3. Solche Methoden sind dem Fachmann bekannt und werden beispielsweise beschrieben in

Houben-Weyl, Methoden der organischen Chemie 4. Auflage, Organo-Stickstoff-Verbindungen I, Teil 2, Georg Thieme Verlag Stuttgart 1990, S. 1087-1136 sowie in den dort zitierten Literaturstellen. Beispielsweise kann ein Anilin der Formel XI über den Weg der Diazotierung-Substitution in ein Sulfochlorid der Formel XII (R3 = SO₂Cl) überführt werden, wie beispielsweise beschrieben in Houben-Weyl, Methoden der organischen Chemie 4. Auflage, Organo-Schwefel-Verbindungen, Teil 2, Georg Thieme Verlag Stuttgart 1985, S. 1069-1070.

In Schritt k werden die Verbindungen der Formel XII nach dem Fachmann bekannten Methoden und wie oben beschrieben zu den Verbindungen der Formel II derivatisiert. In diesem Schritt können beispielsweise die Sulfochloride der Formel XII (R3 = SO₂Cl) zunächst zu den entsprechenden Sulfinsäuren (wie zum Beispiel in Houben-Weyl, Methoden der organischen Chemie 4. Auflage, Organo-Schwefel-Verbindungen, Teil 1, Georg Thieme Verlag Stuttgart 1985, S. 620-621 und Houben-Weyl, Methoden der organischen Chemie, Schwefel-, Selen-, Tellur-Verbindungen, Georg Thieme Verlag Stuttgart 1955, S.304-309 beschrieben) umgesetzt und anschließend zum Methylsulfon alkyliert werden, wie zum Beispiel beschrieben in Houben-Weyl, Methoden der organischen Chemie 4. Auflage, Organo-Schwefel-Verbindungen, Teil 2, Georg Thieme Verlag Stuttgart 1985, S. 1145-1149. Dabei tritt gleichzeitige Veresterung der Carbonsäure zum Methylester ein.

Für die Darstellung der Verbindungen der Formel 1, in denen R1 Wasserstoff ist, erfolgt die Synthese ohne die Schritte b und c.
Für die Darstellung der Verbindungen der Formel 1, in denen R3 NR9R10 ist, erfolgt die Synthese ohne den Schritt i.
In den Ausgangsverbindungen können auch funktionelle Gruppen in geschützter Form oder in Form von Vorstufen vorliegen und dann in den nach dem oben beschriebenen Verfahren hergestellten Verbindungen der Formel II in die gewünschten Gruppen überführt werden. Entsprechende Schutzgruppentechniken sind dem Fachmann bekannt.
Ebenso können entsprechende funktionelle Gruppen nach dem Fachmann bekannten Methoden derivatisiert werden. Beispielsweise können Verbindungen, in denen R3 NH₂ ist, durch Umsetzung mit entsprechenden Alkylhalogeniden oder 2,2,2-Trifluorethylhalogeniden, zum Beispiel Methyliodid, Ethyliodid oder 2,2,2-Trifluorethyliodid, zu Verbindungen umgesetzt werden, in denen R3 NR9R10 ist, wobei R9 und R10 unabhängig voneinander Wasserstoff, Alkyl mit 1, 2, 3 oder 4 C-Atomen oder -CH₂-CF₃ und nicht beide gleichzeitig Wasserstoff sind.

Pentafluorsulfanyl-benzoylguanidine der Formel I sind im allgemeinen schwache Basen und können Säure unter Bildung von Salzen binden. Als Säureadditionssalze kommen Salze aller pharmazeutisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formel I sind substituierte Acylguanidine und inhibieren den zellulären Natrium-Protonen-Antiporter (Na⁺/H⁺-Exchanger, NHE), insbesondere den Subtyp NHE-1.

Aufgrund der NHE-inhibitorischen Eigenschaften eignen sich die Verbindungen der Formel I und/oder deren pharmazeutisch verträgliche Salze zur Prävention und Behandlung von Krankheiten, die durch eine Aktivierung von bzw. durch einen aktivierten NHE verursacht werden, sowie von Krankheiten, die sekundär durch die NHE-bedingten Schädigungen verursacht werden.
Die Verbindungen der Formel I können auch zur Behandlung und Prävention von Krankheiten eingesetzt werden, wobei der NHE nur partiell gehemmt wird, beispielsweise durch Verwendung einer niedrigeren Dosierung.

Da NHE-Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotranspörters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Die erfindungsgemäßen Verbindungen sind von Interesse für Arzneimittel gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und/oder deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und/oder deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und/oder deren pharmazeutisch verträgliche Salze eignen sich somit zur Verbesserung der Therapie mit cardiotoxischen Arzneimitteln.
Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

Die Verbindungen der Formel I und/oder deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydrasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und/oder deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel I und/oder deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und der thrombogenen Selectin-Proteine hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan Rezeptor-Antagonisten, Phosphodiesterase-Inhibitoren, Factor-Vlla-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen sich NHE1-Inhibitoren durch starke inhibierende Wirkung auf die Proliferationen von Zellen, beispielsweise der Fibroblasten-Proliferation und der Proliferation der glatten Gefäßmuskelzellen, aus. Deshalb kommen die Verbindungen der Formel I und/oder deren pharmazeutisch verträgliche Salze als wertvolle Therapeutika für Krankheiten in Frage, bei denen die Proliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch NHE-Inhibitoren die Zellmigration inhibiert wird. Daher kommen die Verbindungen der Formel I und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

NHE1-Inhibitoren zeichnen sich weiterhin durch eine Verzögerung oder Verhinderung von fibrotischen Erkrankungen aus. Verbindungen der Formel I und/oder deren pharmazeutisch verträgliche Salze eignen sich somit als Mittel zur Behandlung von Fibrosen des Herzens, sowie der Lungenfibrose, Leberfibrose, der Nierenfibrose und anderer fibrotischer Erkrankungen. Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und/oder deren pharmazeutisch verträgliche Salze zur Prävention und zur Behandlung des Bluthochdrucks und zur Behandlung von Herz-Kreislauferkrankungen. Dabei können sie allein oder mit einem geeigneten Kombinations- und Formulierungspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromakalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1-Inhibitoren eine signifikante antiphlogistische Wirkung haben und somit als Antiinflammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet. Weiterhin könne die erfindungsgemäßen Verbindungen zur Prävention oder Behandlung von Erkrankungen, die durch Protozoen verursacht werden, eingesetzt werden, wie bei Malaria und der Hühnercoccidiose.

Es wurde außerdem gefunden, dass NHE1-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1-Inhibitoren bezüglich der Beeinflussung der Serumlipidspiegel wertvolle therapeutisch verwertbare Eigenschaften zeigen. So erniedrigen sie signifikant die erhöhte SerumKonzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die NHE1-Inhibitoren zu einer deutlichen Reduktion der durch Stoffwechselanomalien induzierten Infarkte und insbesondere zu einer signifikanten Verminderung der induzierten Infarktgröße und dessen Schweregrades.

Die Verbindungen der Formel und/oder deren pharmazeutisch verträgliche Salze finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und AngiotensinRezeptorantagonisten. Eine Kombination eines NHE-Inhibitors der Formel 1 und/oder deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und/oder deren pharmazeutisch verträgliche Salze steigert, erweist sich als günstige Kombination mit verstärkter Wirkung und vermindertem Wirkstoffeinsatz. So führen Verbindungen der Formel I und/oder deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass NHE1-Inhibitoren geeignet in der Behandlung des nicht-insulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I und/oder deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

NHE1-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nicht-ischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und/oder deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und/oder deren pharmazeutisch verträgliche Salze eignen sich somit hervorragend zur Behandlung und Prävention der Herzinsuffizienz, des congestive heart failure (CHF).

Durch Proliferationshemmung können nicht nur die bereits eingetretene Krebserkrankung geheilt werden, sondern auch die altersbedingte Entstehungshäufigkeit von Krebs durch NHE-Inhibitoren vermindert und hochsignifikant verzögert werden. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und/oder deren pharmazeutisch verträgliche Salze eignen sich somit zur Behandlung und insbesondere der Prävention von altersbedingten Formen von Krebs.

Mit NHE-Inhibitoren wird eine zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitszüglern erfolgen. Ebenso kann eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden.

Die Verbindungen der Formel I und/oder deren pharmazeutisch verträgliche Salze eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung enthaltend eine wirksame Menge einer Verbindung der Formel I und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.
Arzneimittel, die eine Verbindung der Formel I und/oder deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel 1 und/oder deren pharmazeutisch verträgliche Salze in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke.und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, zum Beispiel bis zu 4 Einzeldosen pro Tag. Insbesondere bei i.v. Anwendung, etwa bei einem Infarktpatienten auf der Intensivstation können beispielsweise bis zu 700 mg pro Tag notwendig werden und können die erfindungsgemäßen Verbindungen durch Infusion verabreicht werden.

### Liste der Abkürzungen:

- ADMET: Absorption - Distribution - Metabolismus - Ausscheidung - Toxikologie
- CDI: Di-imidazol-1-yl-methanon
- dba: Dibenzylideneaceton
- DIP: Diisopropylether
- DIPEA: Diisopropylethylamin
- DME: 1,2-Dimethoxyethan
- DMF: N,N-Dimethylformamid
- DMSO: Dimethylsulfoxid
- EE: Ethylacetat
- eq.: Äquivalent
- HOAc: Essigsäure
- KOtBu: Kalium-2-methyl-propan-2-olat
- MeOH: Methanol
- Mp: Schmelzpunkt
- MTB: tert.-Butyl-methylether
- NMP: N-Methyl-2-Pyrrolidon
- OAc: Acetat
- dppf: 1, 1'-Bis-(diphenylphosphino)-ferrocen
- RT: Raumtemperatur
- THF: Tetrahydrofuran
- TMEDA: N,N,N',N'-Tetramethyl-ethan-1,2-diamin

### Experimenteller Teil

### Beispiel 1: N-(5-Methansulfonyl-2-methyl-4-pentafluorosulfanyl-benzoyl)-guanidin

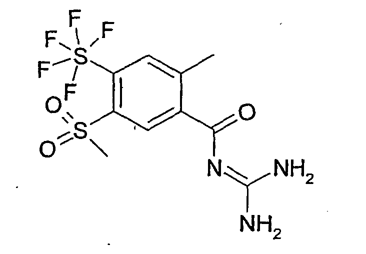

### a) 4-Aminophenyl-schwefelpentafluorid

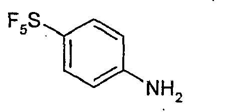

Eine Lösung von Zinn(ll)chlorid (1465 g, 7,73 Mol) in konzentrierter (32-prozentiger) wässriger HCl-Lösung wurde unter Rühren auf 80 °C erwärmt und dann wurde unter Eiskühlung innerhalb von 1 h in 8 Portionen 4-Nitrophenyl-schwefelpentafluorid (584 g, 2,344 Mol) eingetragen. Die Innentemperatur wurde hierbei unter 100 °C gehalten. Anschließend wurde das Gemisch 1,5 h bei 85 °C Innentemperatur gerührt und dann innerhalb einer weiteren Stunde auf 45 °C abkühlen gelassen. Ein Gemisch aus Eis (12 kg), NaOH (2 kg) und Dichlormethan (1,5.L) wurde vorbereitet und das Reaktionsgemisch unter starkem Rühren zugegeben. Die Phasen wurden getrennt, die wässrige Phase wurde 3 x mit je 1 L Dichlormethan extrahiert und die vereinigten organischen Phasen wurden über Na₂SO₄ getrocknet und im Vakuum eingedampft. Man erhielt 510 g 4-Aminophenyl-schwefelpentafluorid als hellgelbes, kristallines Pulver, Mp. 63-65 °C.

### b) 4-Amino-3-brom-phenyl-schwefelpentafluorid

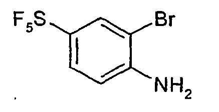

4-Aminophenyl-schwefelpentafluorid (510 g, 2,327 Mol) wurden in Dichlormethan (7 L) gelöst, die Lösung auf 5 °C abgekühlt und unter Rühren 1,3-Dibrom-5,5-dimethylimidazolidin-2,4-dion (326 g, 1,14 Mol) in mehreren Portionen unter Eiskühlung so eingetragen, dass die Innentemperatur bei 3-8 °C gehalten wurde (etwa 1 h). Anschließend wurde das Gemisch ohne äußere Kühlung 1 h rühren und auf Raumtemperatur aufwärmen gelassen. Das Gemisch wurde über ein Kieselgelbett (Volumen etwa 1 L) filtriert, mit Dichlormethan (5,5 L) nachgewaschen und das Filtrat im Vakuum eingedampft. Man erhielt etwa 700 g einer rotbraunen, kristallinen Masse, die bei 60 °C in n-Heptan (600 mL) gelöst wurde und danach im Kühlschrank bei 4 °C kristallisiert. Nach dem Absaugen erhielt man 590 g (85 %) 4-Amino-3-brom-phenylschwefelpentafluorid als bräunliche Kristalle,
Mp. 59-59,5 °C.

### c) 4-Amino-3-methyl-phenyl-schwefelpentafluorid

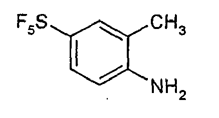

Ein Gemisch aus Cs₂CO₃ (794 g, 2,7 Mol), Dimethoxyethan (2 L), Wasser (300 mL) und Trimethylboroxin (50-prozentige Lösung in THF, 225 g, 0,9 Mol) wurde auf 70 °C erwärmt, PdCl₂(dppf) x CH₂Cl₂ (37 g, 45 mmol) zugegeben und eine Lösung von 4-Amino-3-brom-phenyl-schwefelpentafluorid (270 g, 0,9 Mol) in Dimethoxyethan (400 mL) innerhalb von 2 h unter Erhitzen des Reaktionsgemisches am Rückfluß zugetropft. Anschließend wurde weitere 3 h am Rückfluß erhitzt, dann auf Raumtemperatur abgekühlt, mit MTB (500 mL) verdünnt, über eine Kieselgelsäule (14 x 7 cm, 70-200 µm) filtriert und mit MTB (2500 mL) nachgewaschen. Das Filtrat wurde im Vakuum eingedampft. Man erhielt 490 g einer schwarzen, halbkristallinen Masse, die einer Wasserdampfdestillation unterzogen wurde. Insgesamt wurden 5,5 L Kondensat gesammelt, aus dem das Produkt sich bereits kristallin abschied. Das Kondensat wurde 3 x mit MTB extrahiert, die vereinigten organischen Phasen über Na₂SO₄ getrocknet und im Vakuum eingedampft. Man erhielt 4-Amino-3-methyl-phenylschwefelpentafluorid (181 g, 76 %) als farblose Kristalle, Mp. 65-66 °C,

### d) 4-Brom-3-methyl-phenyl-schwefelpentafluorid

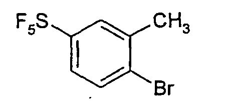

Ein Gemisch aus tert.-Butylnitrit (90-prozentig, 37 ml, 280 mmol) und CuBr₂ (35.8 g, 160 mmol) in Acetonitril (260 mL) wurde bei 5 °C vorgelegt und unter Rühren und Eiskühlung eine Lösung von 4-Amino-3-methyl-phenyl-schwefelpentafluorid (30,9 g, 132,5 mmol) in MTB (140 mL) innerhalb 1 h bei 5-8 °C zugetropft. Dabei setzte nach etwa 2 min Stickstoff-Entwicklung ein. Anschließend wurde das Gemisch innerhalb 1 h unter Rühren auf Raumtemperatur erwärmen gelassen, ein Gemisch aus Eis (250 g), 26-prozentiger wässriger NH₃-Lösung (50 mL) und MTB (250 mL) zugesetzt und das Gemisch 10 min gerührt. Die Phasen wurden getrennt, die wässrigen 3 x mit MTB (je 150 mL) extrahiert und die vereinigten organischen Phasen einmal mit 400 mL Wasser geschüttelt. Nach Trocknen mit Na₂SO₄ und Eindampfen der organischen Phase erhielt man 39 g 4-Brom-3-methyl-phenyl-schwefelpentafluorid als rotbraunes Öl, das mit 8 Mol-% 4,5-Dibrom-3-methyl-phenyl-schwefelpentafluorid verunreinigt war, aber ohne weitere Reinigung weiterverwendet wurde. Ausbeute 89 %, bezogen auf eine Reinheit von 90 %.

### e) 4-Cyan-3-methyl-phenyl-schwefelpentafluorid

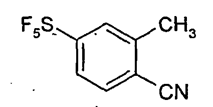

Ein Gemisch aus 4-Brom-3-methyl-phenyl-schwefelpentafluorid (136,4 g, Reinheit 80 %, 0,367 Mol), Zn(CN)₂ (72,8 g, 0,62 Mol) und Zn-Staub (7,2 g, 0,11 Mol) wurde in Dimethylacetamid (900 mL) und Wasser (40 mL) unter Stickstoff-Begasung vorgelegt, unter Rühren auf 125 °C erwärmt und PdCl₂(dppf) x CH₂Cl₂ (32,7 g, 40 mmol) zugesetzt. Nach einstündigem Rühren bei 125 °C wurde nochmals PdCl₂(dppf) x CH₂Cl₂ (16,3 g, 20 mmol) und Zn-Staub (3,6 g, 55 mmol) zugegeben und weitere 2 h bei 125 °C gerührt. Anschließend wurde auf Raumtemperatur abgekühlt, mit n-Heptan (400 mL) verdünnt und das Gemisch unter Zugabe von 5 N wässriger NH₄Cl-Lösung (250 mL) und Wasser (450 mL) 15 min lang stark gerührt. Das Gemisch wurde über eine Kieselgurschicht abgesaugt, die Phasen getrennt und die wässrige 2 x mit n-Heptan (200 mL) extrahiert. Die vereinigten organischen Phasen wurden mit Wasser (450 mL) geschüttelt, über MgSO₄ getrocknet und im Vakuum eingedampft. Der erhaltene schwarze Rückstand wurde in 200 mL n-Heptan gelöst, filtriert und erneut im Vakuum eingedampft. Man erhielt 78 g einer dunkelbraunen Flüssigkeit, die durch Chromatographie an einer Kieselgelsäule (7 x 55 cm, 60-200 µm, n-Heptan/Dichlormethan 4:1 bis 3:2) gereinigt wurde. Als erste Fraktion erhielt man 6,5 g 4-Brom-3-methyl-phenyl-schwefelpentafluorid (Edukt) als gelbliche Flüssigkeit und anschließend 71,1 g (80 %) 4-Cyano-3-methyl-phenyl-schwefelpentafluorid als hellgelbes Öl.

### f) 2-Methyl-4-pentafluorsulfanyl-benzoesäure

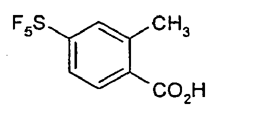

Ein Gemisch aus 4-Cyan-3-methyl-phenyl-schwefelpentafluorid (41,2 g, 169,4 mmol), NaOH (20,4 g, 510 mmol) und Wasser (60 mL) in Ethylenglykol (160 mL) wurde auf 130 °C erwärmt und 4 h bei dieser Temperatur gerührt. Anschließend wurde auf Raumtemperatur abgekühlt, mit MTB (150 mL) und Wasser (250 mL) verdünnt und das Gemisch abgesaugt. Die Phasen des Filtrats wurden getrennt und die wässrige Phase mit konzentrierter wässriger HCl-Lösung angesäuert und der ausfallende Feststoff abgesaugt. Man erhielt 41,1 g (93 %) 2-Methyl-4-pentafluorsulfanylbenzoesäure als farblose Kristalle, Mp. 138-139 °C.

### g) 2-Methyl-5-nitro-4-pentafluorosulfanyl-benzoesäure

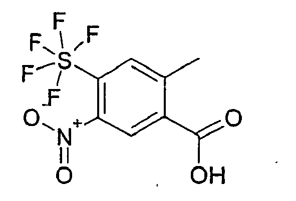

6.0 g 2-Methyl-4-pentafluorsulfanyl-benzoesäure wurden in 60 ml einer 90% wäßrigen HNO₃-Lösung gelöst und bei RT 6 ml einer 96% H₂SO₄ zugetropft. 28 h wurde bei RT stehen gelassen, anschließend auf 300 g Eis gegossen, 300 ml Wasser zugegeben, 1 h nachgerührt und dann das Produkt abfiltriert. An der Luft wurde getrocknet und man erhielt 6.5 g eines blassgelben Feststoffs, Mp. 218-220°C.
R_{f} (DIP/2%HOAc) = 0.27 MS (ES-) : 306

### h) 5-Amino-2-methyl-4-pentafluorosulfanyl-benzoesäure

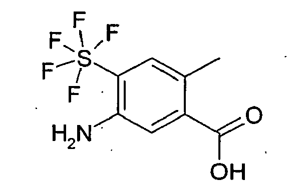

6.5 g 2-Methyl-5-nitro-4-pentafluorsulfanyl-benzoesäure wurden in 100 ml MeOH und 20 ml HOAc gelöst und mit 500 mg 10%Pd/C versetzt. 20 h lang wurde unter Wasserstoff bei Normaldruck und RT hydriert. Die Umsetzung war unvollständig, daher wurde weitere 48 h bei 6 bar Wasserstoffdruck und RT hydriert. Anschließend wurde der Katalysator abfiltriert und die Solventien im Vakuum entfernt. Man erhielt 5.7 g eines hellgrauen Feststoffs, Mp 187-189°C.
R_{f} (DIP/2%HOAc) = 0.23 MS (ES-) : 276

### i) 5-Chlorsulfonyl-2-methyl-4-pentafluorsulfanyl-benzoesäure

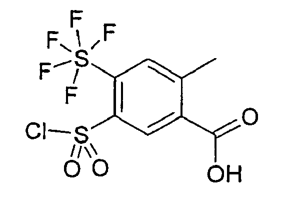

1,0 g 5-Amino-2-methyl-4-pentafluorsulfanyl-benzoesäure wurde in 30 ml HOAc gelöst und mit 30 g Eis und 30 ml einer gesättigten wäßrigen HCl-Lösung versetzt. Anschließend wurde bei 0°C eine Lösung von 274 mg NaNO₂ in 1 ml Wasser innerhalb einer Minute zugetropft. 15 Minuten lang wurde bei 0°C nachgerührt. Dann wurde die so erhaltene Suspension zu einer 0°C kalten Suspension aus 6.1 mg CuCl und 61.5 mg CuCl₂ x 2 H₂O in 30 ml einer gesättigten Lösung von SO₂ in HOAc portionsweise addiert. 1 h wurde bei 0°C, anschließend 1 h bei RT nachgerührt. Anschließend wurde das Reaktionsgemisch 3 mal mit je 200 ml Diethylether extrahiert. Über MgSO₄ wurde getrocknet und die flüchtigen Bestandteile im Vakuum entfernt. Man erhielt 1.3 g des Produktes, das direkt weiter umgesetzt wurde.

### k) 2-Methyl-5-sulfino-4-pentafluorsulfanyl-benzoesäure

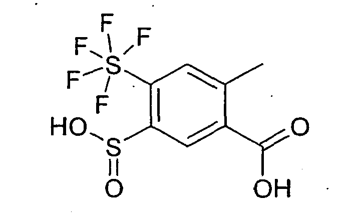

1.2 g 5-Chlorsulfonyl-2-methyl-4-pentafluorsulfanyl-benzoesäure wurden portionsweise zu einer 70°C warmen Lösung von 4.2 g Na₂SO₃ in 50 ml Wasser zugegeben und dabei mit einer 2 N wäßrigen NaOH-Lösung der pH der Lösung zwischen pH=9 und pH=11 gehalten. 20 Minuten wurde bei 70°C nachgerührt, auf RT abgekühlt und mit einer wäßrigen HCI-Lösung auf pH=1-2 gestellt. 16 h wurde bei RT stehen gelassen, anschließend das Produkt abfiltriert und im Vakuum getrocknet. Man erhielt 1.0 g eines weißen Feststoffs, Mp. 288-290°C (unter Zersetzung).
R_{f} (EE/MeOH 1:1) = 0.52

### 1) 5-Methansulfonyl-2-methyl-4-pentafluorsulfanyl-benzoesäure-methylester

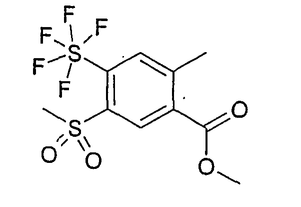

1.0 g 2-Methyl-5-sulfino-4-pentafluorosulfanyl-benzoesäure wurde in 10 ml Wasser suspendiert und 3.1 ml einer wäßrigen 2 N NaOH-Lösung zugegeben (Phenolphthalein: Basisch). Das Wasser wurde im Vakuum entfernt und anschließend 2 mal mit je 20 ml Toluol coevaporiert. Das Dinatriumsalz wurde anschließend in 40 ml wasserfreiem DMF gelöst, 0.69 ml Methyliodid zugegeben und zunächst 4 h bei 60°C, dann 15 h bei RT gerührt. Das Reaktionsgemsch wurde auf 100 ml Wasser gegossen und ein erster Teil des Produktes (500 mg) abgesaugt. Das Filtrat wurde mit wäßriger HCI-Lösung auf pH=2 eingestellt und 3 mal mit je 30 ml EE extrahiert. Über MgSO₄ wurde getrocknet und das Solvens im Vakuum entfernt. Chromatographie an Kieselgel mit DIP lieferte weitere 460 mg weißer Kristalle, Mp 127°C.
R_{f} (DIP) = 0.36

### m) N-(5-Methansulfonyl-2-methyl-4-pentafluorsulfanyl-benzoyl)-guanidin

0.70 g Guanidinium-chlorid und 0.68 g KO*t*Bu wurden in 20 ml wasserfreiem DMF 30 Minuten bei RT gerührt. Diese Suspension wurde dann zu 0.43 g 5-Methansulfonyl-2-methyl-4-pentafluorsulfanyl-benzoesäure-methylester gegeben und 16 h bei RT gerührt. Anschließend wurde das Reaktionsgemisch auf 200 ml Wasser gegossen, mit wäßriger HCl-Lösung auf pH=8 eingestellt und 3 mal mit je 100 ml EE extrahiert. Über MgSO₄ wurde getrocknet und das Solvens im Vakuum entfernt. Der Rückstand wurde in 5 ml CH₂Cl₂ suspendiert und das Produkt abfiltriert. Man erhielt 190 mg farbloser Kristalle, Mp. 254-256°C.
R_{f} (EE) = 0.22 MS (ES⁺) : 382

### Beispiel 2: N-(5-Methansulfonyl-2-methyl-4-pentafluorosulfanyl-benzoyl)-guanidin, Methansulfonsäuresalz

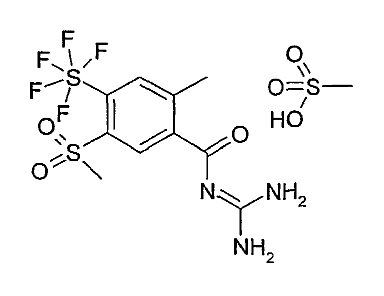

9.3 g der Titelverbindung des Beispiels 1 wurden in 100 ml Wasser suspendiert und eine Lösung von 2.3 g Methansulfonsäure in 10 ml Wasser zugegeben. 30 Minuten wurde bei RT nachgerührt, anschließend das Wasser im Vakuum entfernt. Man erhielt 11,7 g des Methansulfonsäuresalzes, das anschließend noch aus 110 ml Wasser umkristallisiert wurde. Man erhielt 10.0 g N-(5-Methansulfonyl-2-methyl-4-pentafluorosulfanyl-benzoyl)-guanidin, Methansulfonsäuresalz als weiße Kristalle, Mp 230°C.

### Beispiel 3: N-(5-Methansulfonyl-2-methyl-4-pentafluorosulfa,nyl-benzoyl)-guanidin, Hydrochlorid

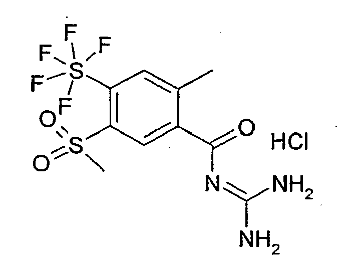

300 mg der Titelverbindung des Beispiels 2 wurden in 50 ml einer gesättigten wäßrigen Na₂CO₃-Lösung suspendiert und 2 mal mit je 40 ml EE extrahiert. Die EE-Phase wurde anschließend über MgSO₄ getrocknet und das Solvens im Vakuum entfernt. Der Rückstand wurde in 10 ml MeOH gelöst und mit 2 ml einer 10% wäßrigen HCl-Lösung versetzt. Die flüchtigen Bestandteile wurden im Vakuum entfernt und man erhielt 230 mg weißer Kristalle, Mp 276-278°C.

### Bestimmung der NHE-Hemmung

Die Hemmkonzentration IC₅₀ für die NHE-1 Hemmung wurde wie folgt bestimmt:

IC₅₀ für die NHE-1 Hemmnung wurden bestimmt in einem FLIPR-Assay mittels Messung der pHᵢ-Erholung in transfizierten Zelllinien, die den humanen NHE-1 exprimieren.

Der Assay wurde im FLIPR (Fluorometric imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließerider Selektion keine endogene NHE-Aktivität auf), wurden am Vortag mit einer Dichte von ~25.000 Zellen /Well ausgesät. Das Wachstumsmedium der transfizierten Zellen (Iscove +10 % fötales Kälberserum) enthielt zusätzlich G418 als Selektionsantibiotikum, um die Anwesenheit der transfizierten Sequenzen sicherzustellen.

Der eigentliche Assay begann mit der Entfernung des Wachstumsmediums und Zugabe von 100 µl/Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchlorid, 1 mM MgCl₂, 1 mM CaCl₂, 5 mM KCI, 20 mM HEPES, 5 mM Glucose; pH 7,4 [mit KOH eingestellt]). Die Zellen wurden dann 20 Minuten bei 37°C inkubiert. Diese Inkubation führte zur Beladung der Zellen mit dem fluoreszierenden Farbstoff, dessen Fluoreszenzintensität vom pHi abhängt, und mit NH₄Cl, was zu einer leichten Alkalinisierung der Zellen führte.
Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.

Nach dieser 20-minütigen Inkubation wurde der Beladungspuffer, der NH₄Cl und freies BCECF-AM enthielt, durch dreimaliges Waschen im Zellwasher (Tecan Columbus) mit jeweils 400 µl Waschpuffer (133,8 mM Cholinchlorid, 4,7 mM KCI, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen betrug 90 µl (50-125 µl möglich). Dieser Waschschritt entfernte das freie BCECF-AM und führte als Folge der Entfernung der externen NH₄⁺-Ionen zu einer intrazellulären Ansäuerung (~ pHi 6.3 - 6.4).

Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wurde, führte der Waschprozess dazu, dass intrazellulär H⁺ zurückblieb, was die Ursache für die intrazelluläre Ansäuerung war. Diese kann letztlich zum Zelltod führen, wenn sie lang genug anhält. An dieser Stelle war es wichtig, dass der Waschpuffer natriumfrei (<1 mM) war, da extrazelluläre Natrium-Ionen zu einer augenblicklichen Erholung des pHᵢ durch die Aktivität der klonierten NHE-Isoformen führen würde.
Es war ebenfalls wichtig, dass alle verwendeten Puffer (Beladungspuffer, Waschpuffer, Recoverypuffer) keine HCO₃⁻-Ionen enthielten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen wurden dann (bis zu 20 Minuten nach der Ansäuerung) zum FLIPR transferiert. Im FLIPR wurde der intrazelluläre Fluoreszenzfarbstoff durch Licht mit einer Wellenlänge von 488 nm, das von einem Argon-Laser erzeugt wurde, angeregt, und die Messparameter (Laserleistung, Belichtungszeit und Blende der im FLIPR eingebauten CCD-Kamera) wurden so gewählt, dass das durchschnittliche Fluoreszenzsignal pro Well zwischen 30000 und 35000 relativen Fluoreszenzeinheiten lag.

Die eigentliche Messung im FLIPR begann damit, dass Software-gesteuert alle zwei Sekunden eine Aufnahme mit der CCD-Kamera gemacht wurde. Nach zehn Sekunden wurde die Erholung des intrazellulären pH's durch Zugabe von 90 µl Recoverypuffer (133,8 mM NaCl, 4,7 mM KCl, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienten Wells, denen reiner Recoverypuffer zugegeben wurde, Negativkontrollen (0 % NHE-Aktivität) erhielten Waschpuffer. In allen anderen Wells wurde Recoverypuffer mit der zweifach konzentrierten Testsubstanz hinzugegeben. Die Messung im FLIPR endete nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear war, sondern am Ende aufgrund abnehmender NHE-Aktivität bei höheren pHᵢ-Werten abfiel, war es wichtig, für die Auswertung der Messung den Teil auszuwählen, in dem die Fluoreszenzzunahme der Positivkontrollen linear war.

| Beispiel | NHE1-Hemmung IC₅₀ [nM] |
|---|---|
| 1 | 49 |

### In vivo Pharmakokinetik - Profilierung mit der "n in one Methode"

Als pharmakokinetische Kenndaten wurden die Expositionsdaten und die Verteilungsvolumina wie folgt bestimmt:

Der erfindungsgemäße NHE-1 Inhibitor des Beispiels 1 und als Referenzsubstanz der bekannte NHE-1 Hemmer Cariporid mit der Formel 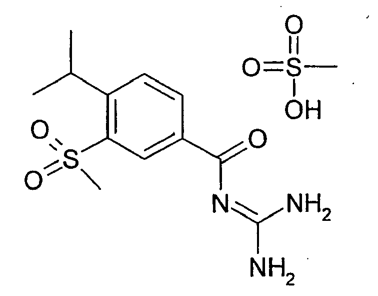 wurden in wässrigem, leicht saurem Medium (Wasser, pH 4, eingestellt mit 1 M Salzsäure) gelöst. Die Konzentration der so hergestellten wässrigen Formulierung betrug ca. 1,5 mg je Substanz pro 1 g Lösung. 10 ml dieser Formulierung wurden einem männlichen, nüchternen Beagle-Hund in die Vena jugularis mittels Katheter als Bolus einmal appliziert (Dosis ca. 1 mg je applizierter Substanz pro kg Körpergewicht des Hundes). Mittels eines zweiten Katheters wurden nach 5min, 15min, 30min, 1 h, 2h, 4h, 8h und 24 h Blutproben gewonnen und heparinisiertes Plasma durch Zentrifugation bei 1000G in entsprechenden Plasmaröhrchen hergestellt.

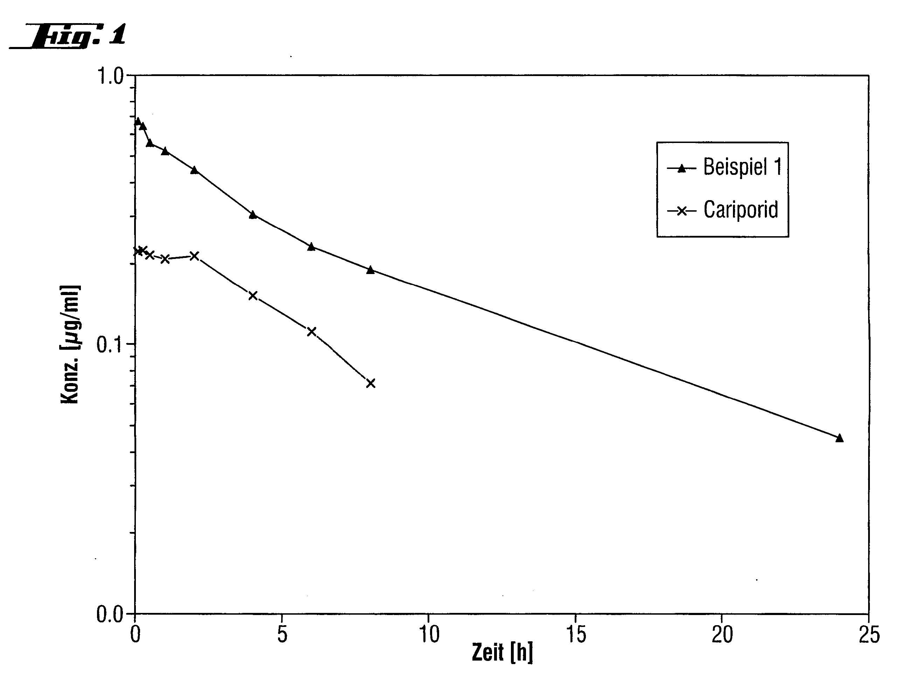

Die Plasmaproben wurden aufgearbeitet und nach einer HPLC-Trennung mittels MS/MS quantifiziert. Diese Methode erlaubte wegen ihrer hohen Spezifität die gleichzeitige Bestimmung mehrerer Substanzen. Aus den Konzentrations - Zeitkurven (siehe Figur 1) ließen sich die Expositionen mittels des Computerprogramms WinNonlin berechnen und mit der Exposition der bekannten NHE-1 Referenzsubstanz vergleichen. Da die verschiedenen Substanzen im gleichen Tier zum gleichen Zeitpunkt gemessen wurden, ergab sich ein exakter Vergleich der Verbindungen und es konnte ein Ranking der Verteilungsvolumina erstellt werden.

| Verbindung | Verteilungsvolumen [l/kg Körpergewicht] |
|---|---|
| Beispiel 1 | 1.67 |
| Referenzsubstanz Cariporid | 2.94 |

Aus den Konzentrations-Zeitkurven in Figur 1 ist deutlich ersichtlich, das die erfindungsgemäße Verbindung im Blut auch über einen längeren Zeitraum erhalten bleibt und damit die Exposition etwa 2-3 mal höher ist als bei der Referenzsubstanz Cariporid. Nach 24 Stunden ist Cariporide im Plasma nicht mehr detektierbar.

In der Figur wurden folgende Beschriftungen und Kennzeichnungen vorgenommen:
Fig. 1: Konzentrations - Zeitkurven im Blutplasma von Hunden nach Gabe von je ca. 1 mg/kg von der Verbindung des Beispiels 1 und von Cariporid.
Y-Achse: Konzentration der gemessenen Verbindung in µg/ml im Plasma
X-Achse: Zeit in h

## Patentansprüche

1. Verbindungen der Formel I 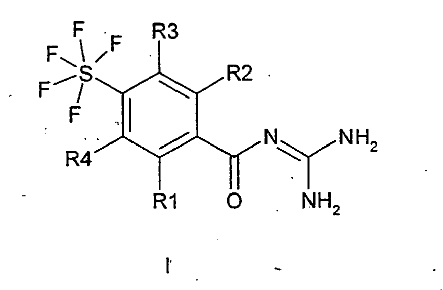 worin bedeuten
R1 Wasserstoff, Alkyl mit 1, 2, 3 oder 4 C-Atomen, Alkoxy mit 1, 2, 3 oder 4 C Atomen, F, Cl, Br, I, -CN, NR5R6, -Oₚ-(CH₂)ₙ-(CF₂)ₒ-CF₃ oder -(SOₘ)_{q}-(CH₂)ᵣ-(CF₂)ₛ-CF₃;
R5 und R6 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
R2 Wasserstoff, Alkyl mit 1, 2, 3 oder 4 C-Atomen, Alkoxy mit 1, 2, 3 oder 4 C-Atomen, F, Cl, Br, I, -CN, NR7R8, -Oₜ-(CH₂)ᵤ-(CF₂)ᵥ-CF₃ oder -(SO_{w})ₓ-(CH₂)_{y}-(CF₂)_{z}-CF₃ ;
R7 und R8 unabhängig voneinander Wasserstoff, Alkyl mit 1, 2, 3 oder 4 C-Atomen oder -CH₂-CF₃;
w Null, 1 oder 2
t, u, v, x, y und z unabhängig voneinander Null oder 1;
R3 Cl, Br, I, -CN, -SO₂CH₃, Alkoxy mit 1, 2, 3 oder 4 C-Atomen, NR9R10, -Oₐ-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
a, b und c unabhängig voneinander Null oder 1;
d Null, 1 oder 2;
e Null oder 1;
f Null, 1, 2, 3 oder 4;
g Null oder 1;
oder
R3 -(CH₂)ₕ -Phenyl oder -O-Phenyl,
in denen die Phenylreste unsubstituiert sind oder substituiert sind mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
h Null, 1, 2, 3 oder 4;
oder
R3 -(CH₂)ₐₐ -Heteroaryl,
das unsubstituiert ist oder substituiert ist mit 1, 2 oder 3 Resten ausgewählt , aus der Gruppe bestehend aus F, Cl, Br, l, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
aa Null, 1, 2, 3 oder 4;
R4 Wasserstoff, F, Cl, Br, I, -CN, -SO₂CH₃, Alkoxy mit 1, 2, 3 oder 4 C-Atomen, NR11R12, -O_{dd}-(CH₂)ₑₑ-(CF₂)_{ff}-CF₃, -(SO_{gg})ₕₕ-(CH₂)ⱼⱼ-(CF₂)ₖₖ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R11 und R12 unabhängig voneinander Wasserstoff, Alkyl mit 1, 2, 3 oder 4 C-Atomen oder -CH₂-CF₃;
dd, ee und ff unabhängig voneinander Null oder 1;
gg Null, 1 oder 2;
hh Null oder 1;
jj Null, 1, 2, 3 oder 4;
kk Null oder 1;
oder
R4 -(CH₂)ₗₗ -Phenyl oder -O-Phenyl,
in denen die Phenylreste unsubstituiert sind oder substituiert sind mit 1, 2 oder 3 Resten ausgewählt aus der Gruppe bestehend aus F, Cl, Br, l, -Oₘₘ-(CH₂)ₙₙ-CF₃, Alkoxy mit 1, 2, 3 oder 4 C-Atomen, Alkyl mit 1, 2, 3 oder 4 C-Atomen und -SO₂CH₃;
mm Null oder 1;
nn Null, 1; 2 oder 3;
ll Null, 1, 2, 3 oder 4;
oder
R4 -(CH₂)ₒₒ -Heteroaryl,
das unsubstituiert ist oder substituiert ist mit 1, 2 oder 3 Resten ausgewählt aus der Gruppe bestehend aus F, Cl, Br, I, -Oₚₚ-(CH₂)ᵣᵣ-CF₃, Alkoxy mit 1, 2, 3 oder 4 C-Atomen, Alkyl mit 1, 2, 3 oder 4 C-Atomen und -SO₂CH₃;
pp Null oder 1;
rr Null, 1, 2 oder 3;
oo Null, 1, 2, 3 oder 4;
sowie deren pharmazeutisch verträgliche Salze.

2. Verbindungen der Formel I nach Anspruch 1, in denen bedeuten:
R1 Wasserstoff, Alkyl mit 1, 2, 3 oder 4 C-Atomen, Alkoxy mit 1, 2, 3 oder 4 C-Atomen, F, Cl, Br, I, -CN, NR5R6, -Oₚ-(CH₂)ₙ-(CF₂)ₒ-CF₃ oder -(SOₘ)_{q}-(CH₂)ᵣ-(CF₂)ₛ-CF₃;
R5 und R6 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
R2 Wasserstoff oder F;
R3 Cl, Br, I, -CN, -SO₂CH₃, Alkoxy mit 1, 2, 3 oder 4 C-Atomen, NR9R10, -Oₐ-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
a, b und c unabhängig voneinander Null oder 1;
d Null, 1 oder 2;
e Null oder 1;
f Null, 1, 2, 3 oder 4;
g Null oder 1;
oder
R3 -(CH₂)ₕ -Phenyl oder -O-Phenyl,
in denen die Phenylreste unsubstituiert sind oder substituiert sind mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
h Null, 1, 2, 3 oder 4;
oder
R3 -(CH₂)ₐₐ -Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃ ;
bb Null oder 1 ;
cc Null, 1, 2 oder 3;=
aa Null, 1, 2, 3 oder 4;
R4 Wasserstoff oder F;
sowie deren pharmazeutisch verträgliche Salze.

3. Verbindungen der Formel I nach Anspruch 1 oder 2, in denen bedeuten:
R1 Wasserstoff, Alkyl mit 1, 2, 3 oder 4 C-Atomen, Alkoxy mit 1, 2, 3 oder 4 C-Atomen, F, Cl, Br, 1, -CN, NR5R6, -O-CH₂-CF₃ oder -(SOₘ)_{q}-(CH₂)ᵣCF₃;
R5 und R6 unabhängig voneinander Wasserstoff, Alkyl mit 1, 2, 3 oder 4 C-Atomen oder -CH₂-CF₃;
m Null, 1 oder 2
q und r unabhängig voneinander Null oder 1;
R2 Wasserstoff oder F;
R3 Cl, Br, I, -CN, -SO₂CH₃, Alkoxy mit 1, 2, 3 oder 4 C-Atomen, NR9R10, -O-CH₂-CF₃, -(SO_{d})ₑ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
d Null, 1 oder 2;
e Null oder 1;
oder
R3 Phenyl,
das unsubstituiert ist oder substituiert ist mit 1, 2 oder 3 Resten ausgewählt aus der Gruppe bestehend aus F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
oder
R3 Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1 ;
cc Null, 1, 2 oder 3;
R4 Wasserstoff oder F;
sowie deren pharmazeutisch verträgliche Salze.

4. Verbindungen der Formel I nach einem oder mehreren der Ansprüche 1, 2 oder 3, in denen bedeuten:
R1 Wasserstoff, Alkyl mit 1, 2, 3 oder 4 C-Atomen, Methoxy, Ethoxy, F, Cl, NR5R6, -O-CH₂-CF₃ oder -(SOₘ)_{q}-(CH₂)ᵣ-CF₃ ;
R5 und R6 unabhängig voneinander Wasserstoff, Alkyl mit 1, 2, 3 oder 4 C-Atomen oder -CH₂-CF₃;
m Null, 1 oder 2
q und r unabhängig voneinander Null oder 1;
R2 Wasserstoff oder F;
R3 Cl, -CN, -SO₂CH₃, Methoxy, Ethoxy, NR9R10, -O-CH₂-CF₃, -(SO_{d})ₑ-CF₃, Alkyl mit 1, 2, 3, 4, 5 oder 6 C-Atomen oder Cycloalkyl mit 3, 4, 5, 6 oder 7 C-Atomen, in dem 1, 2, 3 oder 4 Wasserstoff-Atome durch Fluoratome ersetzt sein können;
R9 und R10 unabhängig voneinander Wasserstoff, Methyl, Ethyl oder -CH₂-CF₃;
d Null, 1 oder 2;
e Null oder 1;
oder
R3 Phenyl,
das unsubstituiert ist oder substituiert ist mit 1 oder 2 Resten ausgewählt aus der Gruppe bestehend aus F, Cl, -Oⱼ-(CH₂)ₖ-CF₃, Methoxy, Ethoxy, Alkyl mit 1, 2, 3 oder 4 C-Atomen und -SO₂CH₃;
j und k unabhängig voneinander Null oder 1;
oder
R3 Heteroaryl,
das unsubstituiert ist oder substituiert ist mit 1 oder 2 Resten ausgewählt aus der Gruppe bestehend aus F, Cl, -O_{bb}-(CH₂)_{cc}-CF₃, Methoxy, Ethoxy, Alkyl mit 1, 2, 3 oder 4 C-Atomen und -SO₂CH₃;
bb und cc unabhängig voneinander Null oder 1;
R4 Wasserstoff oder F;
sowie deren pharmazeutisch verträgliche Salze.

5. Verbindungen der Formel I nach einem der Ansprüche 1 bis 4 ausgewählt aus: N-(5-Methansulfonyl-2-methyl-4-pentafluorosulfanyl-benzoyl)-guanidin, sowie dessen pharmazeutisch verträgliche Salze.

6. Verfahren zur Herstellung einer Verbindung der Formel I nach einem oder : mehreren der Ansprüche 1, 2, 3, 4 oder 5 oder deren pharmazeutisch verträgliche Salze, **dadurch gekennzeichnet, dass** man eine Verbindung der Formel II, 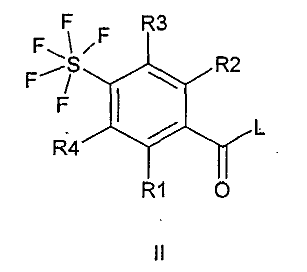 worin R1 bis R4 die in den Ansprüchen 1 bis 4 angegebene Bedeutung besitzen und L für eine nucleophil substituierbare Fluchtgruppe steht, mit Guanidin umsetzt.

7. Verbindung der Formel I oder deren pharmazeutisch verträgliche Salze nach einem oder mehreren der Ansprüche 1 bis 5 zur Verwendung als Medikament.

8. Verwendung einer Verbindung der Formel I oder deren pharmazeutisch verträgliche Salze nach einem oder mehreren der Ansprüche 1 bis 5 zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden; von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

9. Verwendung einer Verbindung der Formel I oder deren pharmazeutisch verträgliche Salze nach einem oder mehreren der Ansprüche 1 bis 5 in Kombination mit anderen Arzneimitteln oder Wirkstoffen zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata- Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

10. Verwendung einer Verbindung der Formel I oder deren pharmazeutisch verträgliche Salze nach Anspruch 9 in der Kombination mit cardiotoxischen und cytotoxischen Arzneimitteln oder Wirkstoffen zur Herstellung eines Medikaments mit verminderten cardiotoxischen und cytotoxischen Eigenschaften.

11. Verwendung einer Verbindung der Formel I oder deren pharmazeutisch verträgliche Salze allein oder in Kombination mit anderen Arzneimitteln oder Wirkstoffen nach Anspruch 8 und/oder 9 zur Herstellung eines Medikaments zur Behandlung oder Prophylaxe von akuten oder chronischen Schäden, Erkrankungen oder indirekten Folgeerkrankungen von Organen und Geweben, die durch lschämie- oder durch Reperfusionsereignisse verursacht werden.

12. Verwendung einer Verbindung der Formel I oder deren pharmazeutisch verträgliche Salze allein oder in Kombination mit anderen Arzneimitteln oder Wirkstoffen nach Anspruch 8 und/oder 9 zur Herstellung eines Medikaments zur Behandlung lebensbedrohlichen Kammerflimmerns des Herzens.

13. Verwendung einer Verbindung der Formel I oder deren pharmazeutisch verträgliche Salze allein oder in Kombination mit anderen Arzneimitteln oder Wirkstoffen nach Anspruch 8 und/oder 9 zur Herstellung eines Medikaments zur Behandlung oder zur Prophylaxe der Metastasierung.

14. Verwendung einer Verbindung der Formel I oder deren pharmazeutisch verträgliche Salze allein oder in Kombination mit anderen Arzneimitteln oder Wirkstoffen nach Anspruch 8 und/oder 9 zur Herstellung eines Medikaments zur Behandlung oder Prophylaxe fibrotischer Erkrankungen des Herzens, der Herzinsuffizienz oder des Congestive Heart failure.

15. Heilmittel für die humane, veterinäre oder phytoprotektive Anwendung enthaltend eine wirksame Menge einer Verbindung der Formel I und/oder deren pharmazeutisch verträgliche Salze nach einem oder mehreren der Ansprüche 1 bis 5, zusammen mit pharmazeutisch annehmbaren Träger- und Zusatzstoffen.

16. Heilmittel für die humane, veterinäre oder phytoprotektive Anwendung enthaltend eine wirksame Menge einer Verbindung der Formel I oder deren pharmazeutisch verträgliche Salze nach einem oder mehreren der Ansprüche 1 bis 5, zusammen mit pharmazeutisch annehmbaren Träger- und Zusatzstoffen in Kombination mit anderen pharmakologischen Wirkstoffen oder Arzneimitteln.

## Claims

1. A compound of the formula I 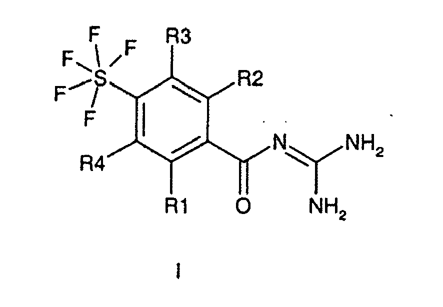 in which
R1 is hydrogen, alkyl having 1, 2, 3 or 4 carbon atoms, alkoxy having 1, 2, 3 or 4 carbon atoms, F, Cl, Br, I, -CN, NR5R6, -Oₚ-(CH₂)ₙ-(CF₂)ₒ CF₃ or -(SOₘ)_{q} -(CH₂)ᵣ-(CF₂)ₛ-CF₃;
R5 and R6 are, independently of one another, hydrogen, alkyl having 1, 2, 3 or 4 carbon atoms or -CH₂-CF₃;
m is zero, 1 or 2
n, o, p, q, r and s independently of one another, zero or 1;
R2 are, hydrogen, alkyl having 1, 2, 3 or 4 carbon atoms, alkoxy having 1, 2, 3 or 4 carbon atoms, F, Cl, Br, I, -CN, NR7R8, -Oₜ-(CH₂)ᵤ-(CF₂)ᵥ-CF₃ or -(SO_{w})ₓ (CH₂)_{y}-(CF₂)_{z}-CF₃;
R7 and R8 are, independently of one another, hydrogen, alkyl having 1, 2, 3 or 4 carbon atoms or -CH₂-CF₃;
w is zero, 1 or 2
t, u, v, x, y and z are, independently of one another, zero or 1;
R3 is Cl, Br, I, -CN, -SO₂CH₃, alkoxy having 1, 2, 3 or 4 carbon atoms,
NR9R10, -Oₐ-(CH₂)_{b}-(CF₂)_{c}-CF₃, -(SO_{d})ₑ-(CH₂)_{f}-(CF₂)_{g}-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be replaced by fluorine atoms;
R9 and R10 are, independently of one another, hydrogen, alkyl having 1, 2, 3 or 4 carbon atoms or -CH₂-CF₃;
a, b and c are, independently of one another, zero or 1;
d is zero, 1 or 2;
e is zero or 1 ;
f is zero, 1, 2, 3 or 4;
g is zero or 1;
or
R3 is -(CH₂)ₕ-phenyl or -O-phenyl,
in which the phenyl radicals are unsubstituted or substituted by 1, 2 or 3 radicals selected from the group consisting of F, Cl, Br, I, -Oⱼ-(CH₂)ₖ-CF₃, alkoxy having 1, 2, 3 or 4 carbon atoms, alkyl having 1, 2, 3 or 4 carbon atoms and -SO₂CH₃;
j is zero or 1;
k is zero, 1, 2 or 3;
h is zero, 1, 2, 3 or 4;
or
R3 is -(CH₂)ₐₐ-heteroaryl,
which is unsubstituted or substituted by 1, 2 or 3 radicals selected from the group consisting of F, Cl, Br, I, -O_{bb}-(CH₂)_{cc}-CF₃, alkoxy having 1, 2, 3 or 4 carbon atoms, alkyl having 1, 2, 3 or 4 carbon atoms and -SO₂CH₃;
bb is zero or 1;
cc is zero or 1, 2 or 3;
aa is zero, 1, 2, 3 or 4;
R4 is hydrogen, F, Cl, Br, I, -CN, -SO₂CH₃, alkoxy having 1, 2, 3 or 4 carbon atoms, NR11 R12, -O_{dd}-(CH₂)ₑₑ-(CF₂)_{ff}-CF₃; -(SO_{gg})ₕₕ-(CH₂)ⱼⱼ-(CF₂)ₖₖ-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be substituted by fluorine atoms;
R11 and R12 are, independently of one another, hydrogen, alkyl having 1, 2, 3 or 4 carbon atoms or -CH₂-CF₃;
dd, ee and ff are, independently of one another, zero or 1 ;
gg is zero, 1 or 2;
hh is zero or 1;
jj is zero, 1, 2, 3 or 4;
kk is zero or 1;
or
R4 is -(CH₂)ₗₗ-phenyl or -O-phenyl,
in which the phenyl radicals are unsubstituted or substituted by 1, 2 or 3 radicals selected from the group consisting of F, Cl, Br, I, -Oₘₘ-(CH₂)ₙₙ-CF₃, alkoxy having 1, 2, 3 or 4 carbon atoms, alkyl having 1, 2, 3 or 4 carbon atoms and -SO₂CH₃;
mm is zero or 1;
nn is zero, 1, 2 or 3;
ll is zero, 1, 2, 3 or 4;
or
R4 is -(CH₂)ₒₒ-heteroaryl,
which is unsubstituted or substituted by 1, 2 or 3 radicals selected from the group consisting of F, Cl, Br, I, -Oₚₚ-(CH₂)ᵣᵣ-CF₃, alkoxy having 1, 2, 3 or 4 carbon atoms, alkyl having 1, 2, 3 or 4 carbon atoms and -SO₂CH₃;
pp is zero or 1;
rr is zero, 1, 2 or 3;
oo is zero, 1, 2, 3 or 4;
and the pharmaceutically acceptable salts thereof.

2. A compound of the formula I as claimed in claim 1, in which the meanings are:
R1 hydrogen, alkyl having 1, 2, 3 or 4 carbon atoms, alkoxy having 1, 2, 3 or 4 carbon atoms, F, Cl, Br, I, -CN, NR5R6, -Oₚ-(CH₂)ₙ-(CF₂)ₒ-CF₃ or -(SOₘ)_{q}-(CH₂)ᵣ-(CF₂)ₛ-CF₃;
R5 and R6 independently of one another hydrogen, alkyl having 1, 2, 3 or 4 carbon atoms or -CH₂-CF₃;
m zero, 1 or 2
n, o, p, q, r and s independently of one another zero or 1;
R2 hydrogen or F;
R3 Cl, Br, l, -CN, -SO₂CH₃, alkoxy having 1, 2, 3 or 4 carbon atoms,
NR9R10, -Oₐ-(CH₂)_{b}-(CF₂)_{c}-CF₃, -(SO_{d})ₑ-(CH₂)_{f}-(CF₂)_{g}-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be replaced by fluorine atoms;
R9 and R10 independently of one another hydrogen, alkyl having 1, 2, 3 or 4 carbon atoms or -CH₂-CF₃;
a, b and c independently of one another zero or 1;
d zero, 1 or 2;
e zero or 1;
f zero, 1, 2, 3 or 4;
g zero or 1;
or
R3 -(CH₂)ₕ-phenyl or -O-phenyl,
in which the phenyl radicals are unsubstituted or substituted by 1, 2 or 3 radicals selected from the group consisting of F, Cl, Br, I, -Oⱼ-(CH₂)ₖ-CF₃, alkoxy having 1, 2, 3 or 4 carbon atoms, alkyl having 1, 2, 3 or 4 carbon atoms and -SO₂CH₃;
j zero or 1;
k zero, 1, 2 or 3;
h zero, 1, 2, 3 or 4;
or
R3 -(CH₂)ₐₐ-heteroaryl,
which is unsubstituted or substituted by 1, 2 or 3 radicals selected from the group consisting of F, Cl, Br, I, -O_{bb}-(CH₂)_{cc}-CF₃, alkoxy having 1, 2, 3 or 4 carbon atoms, alkyl having 1, 2, 3 or 4 carbon atoms and -SO₂CH₃;
bb zero or 1;
cc zero, 1, 2 or 3;
aa zero, 1, 2, 3 or 4;
R4 hydrogen or F;
and the pharmaceutically acceptable salts thereof.

3. A compound of the formula I as claimed in claim 1 or 2, in which the meanings are:
R1 hydrogen, alkyl having 1, 2, 3 or 4 carbon atoms, alkoxy having 1, 2, 3 or 4 carbon atoms, F, Cl, Br, I, -CN, NR5R6, -O-CH₂-CF₃ or -(SOₘ)_{q}-(CH₂)ᵣ-CF₃;
R5 and R6 independently of one another hydrogen, alkyl having 1, 2, 3 or 4 carbon atoms or -CH₂-CF₃;
m zero, 1 or 2
q and r independently of one another zero or 1;
R2 hydrogen or F;
R3 Cl, Br, l, -CN, -SO₂CH₃, alkoxy having 1, 2, 3 or 4 carbon atoms,
NR9R10, -O-CH₂-CF₃, -(SO_{d})ₑ-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be replaced by fluorine atoms;
R9 and R10 independently of one another hydrogen, alkyl having 1, 2, 3 or 4 carbon atoms or -CH₂-CF₃;
d zero, 1 or 2;
e zero or 1;
or
R3 phenyl,
which is unsubstituted or substituted by 1, 2 or 3 radicals selected from the group consisting of F, Cl, Br, I, -Oⱼ-(CH₂)ₖ-CF₃, alkoxy having 1, 2, 3 or 4 carbon atoms, alkyl having 1, 2, 3 or 4 carbon atoms and -SO₂CH₃;
j zero or 1;
k zero, 1, 2 or 3;
or
R3 heteroaryl,
which is unsubstituted or substituted by 1, 2 or 3 radicals selected from the group consisting of F, Cl, Br, I, -O_{bb}-(CH₂)_{cc}-CF₃, alkoxy having 1, 2, 3 or 4 carbon atoms, alkyl having 1, 2, 3 or 4 carbon atoms and -SO₂CH₃;
bb zero or 1;
cc zero, 1, 2 or 3;
R4 hydrogen or F;
and the pharmaceutically acceptable salts thereof.

4. A compound of the formula I as claimed in one or more of claims 1, 2 or 3, in which the meanings are:
R1 hydrogen, alkyl having 1, 2, 3 or 4 carbon atoms, methoxy, ethoxy, F, Cl, NR5R6, -O-CH₂-CF₃ or -(SOₘ)_{q}-(CH₂)ᵣ-CF₃;
R5 and R6 independently of one another hydrogen, alkyl having 1, 2, 3 or 4 carbon atoms or -CH₂-CF₃;
m zero, 1 or 2
q and r independently of one another zero or 1;
R2 hydrogen or F;
R3 Cl, -CN, -SO₂CH₃, methoxy, ethoxy, NR9R10, -O-CH₂-CF₃, -(SO_{d})ₑ-CF₃, alkyl having 1, 2, 3, 4, 5 or 6 carbon atoms or cycloalkyl having 3, 4, 5, 6 or 7 carbon atoms, in which 1, 2, 3 or 4 hydrogen atoms may be replaced by fluorine atoms;
R9 and R10 independently of one another hydrogen, methyl, ethyl or -CH₂-CF₃;
d zero, 1 or 2;
e zero or 1;
or
R3 phenyl,
which is unsubstituted or substituted by 1 or 2 radicals selected from the group consisting of F, Cl, -Oⱼ-(CH₂)ₖ-CF₃, methoxy, ethoxy, alkyl having 1, 2, 3 or 4 carbon atoms and -SO₂CH₃;
j and k independently of one another zero or 1;
or
R3 heteroaryl,
which is unsubstituted or substituted by 1 or 2 radicals selected from the group consisting of F, Cl, -O_{bb}-(CH₂)_{cc}-CF₃, methoxy, ethoxy, alkyl having 1, 2, 3 or 4 carbon atoms and -SO₂CH₃ ;
bb and cc independently of one another zero or 1;
R4 hydrogen or F;
and the pharmaceutically acceptable salts thereof.

5. A compound of the formula I as claimed in any of claims 1 to 4 and selected from: N-(5-methanesulfonyl-2-methyl-4-pentafluorosulfanylbenzoyl)guanidine, and the pharmaceutically acceptable salts thereof.

6. A process for preparing a compound of the formula I as claimed in one or more of claims 1, 2, 3, 4 or 5 or the pharmaceutically acceptable salts thereof, which comprises reacting a compound of the formula II, 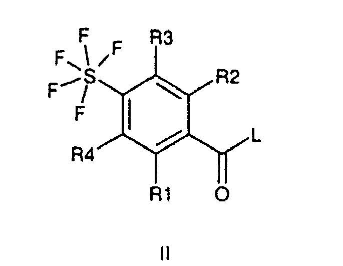 in which R1 to R4 have the meaning stated in claims 1 to 4 and L is a leaving group which can undergo nucleophilic substitution, with guanidine.

7. A compound of the formula I or the pharmaceutically acceptable salts thereof as claimed in one or more of claims 1 to 5 for use as medicament.

8. The use of a compound of the formula I or the pharmaceutically acceptable salts thereof as claimed in one or more of claims 1 to 5 for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for use in bypass operations, for use in resuscitation after a cardiac arrest, for preventing age-related tissue change, for producing a medicament directed against aging or for prolonging life, for the treatment and reduction of the cardiotoxic effects in thyrotoxicosis or for producing a diagnostic aid.

9. The use of a compound of the formula I or the pharmaceutically acceptable salts thereof as claimed in one or more of claims 1 to 5 in combination with other medicaments or active ingredients for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for use in bypass operations, for use in resuscitation after a cardiac arrest, for preventing age-related tissue change, for producing a medicament directed against aging or for prolonging life, for the treatment and reduction of the cardiotoxic effects in thyrotoxicosis or for producing a diagnostic aid.

10. The use of a compound of the formula I or the pharmaceutically acceptable salts thereof as claimed in claim 9 in combination with cardiotoxic and cytotoxic medicaments or active ingredients for producing a medicament with reduced cardiotoxic and cytotoxic properties.

11. The use of a compound of the formula I or the pharmaceutically acceptable salts thereof alone or in combination with other medicaments or active ingredients as claimed in claim 8 and/or 9 for producing a medicament for the treatment or prophylaxis of acute or chronic damage, disorders or indirect sequelae of organs and tissues caused by ischemic or reperfusion events.

12. The use of a compound of the formula I or the pharmaceutically acceptable salts thereof alone or in combination with other medicaments or active ingredients as claimed in claim 8 and/or 9 for producing a medicament for the treatment of life-threatening cardiac ventricular fibrillation.

13. The use of a compound of the formula l or the pharmaceutically acceptable salts thereof alone or in combination with other medicaments or active ingredients as claimed in claim 8 and/or 9 for producing a medicament for the treatment or prophylaxis of metastasis.

14. The use of a compound of the formula I or the pharmaceutically acceptable salts thereof alone or in combination with other medicaments or active ingredients as claimed in claim 8 and/or 9 for producing a medicament for the treatment or prophylaxis of fibrotic disorders of the heart, of heart failure or of congestive heart failure.

15. A medicine for human, veterinary or phytoprotective use comprising an effective amount of a compound of the formula I and/or the pharmaceutically acceptable salts thereof as claimed in one or more of claims 1 to 5, together with pharmaceutically acceptable carriers and additives.

16. A medicine for human, veterinary or phytoprotective use comprising an effective amount of a compound of the formula I and/or the pharmaceutically acceptable salts thereof as claimed in one or more of claims 1 to 5, together with pharmaceutically acceptable carriers and additives in combination with other pharmacological active ingredients or medicaments.

## Revendications

1. Composés de formule I 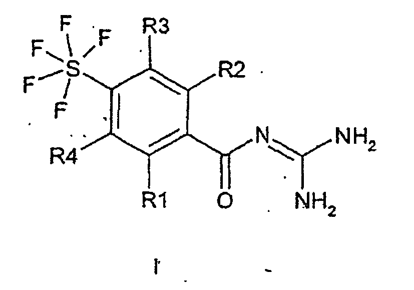 dans laquelle
R1 représente un atome d'hydrogène, un groupe alkyle ayant 1, 2, 3 ou 4 atomes de carbone, alcoxy ayant 1, 2, 3 ou 4 atomes de carbone, F, Cl, Br, I, -CN, NR5R6, -Oₚ-(CH₂)ₙ-(CF₂)ₒ-CF₃ ou -(SOₘ)_{q}-(CH₂)ᵣ-(CF₂)ₛ-CF₃;
R5 et R6 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
n, o, p, q, r et s représentent, indépendamment les uns des autres, zéro ou 1 ;
R2 représente un atome d'hydrogène, un groupe alkyle ayant 1, 2, 3 ou 4 atomes de carbone, alcoxy ayant 1, 2, 3 ou 4 atomes de carbone, F, Cl, Br, l, -CN, NR7R8, -Oₜ-(CH₂)ᵤ-(CF₂)ᵥ-CF₃ ou -(SO_{w})ₓ-(CH₂)_{y}-(CF₂)_{z}-CF₃ ;
R7 et R8 représentent, indépendamment l'un de l'autre, un atome d'hydrogène, un groupe alkyle ayant 1, 2, 3 ou 4 atomes de carbone ou -CH₂-CF₃ ;
w est zéro, 1 ou 2 ;
t, u, v, x, y et z représentent, indépendamment les uns des autres, zéro ou 1 ;
R3 représente Cl, Br, l, -CN, -SO₂CH₃, un groupe alcoxy ayant 1, 2, 3 ou 4 atomes de carbone, NR9R10, -Oₐ-(CH₂)_{b}-(CF₂)_{c}-CF₃, -(SO_{d})ₑ-(CH₂)_{f}-(CF₂)_{g}-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor;
R9 et R10 représentent, indépendamment l'un de l'autre, un atome d'hydrogène, un groupe alkyle ayant 1, 2, 3 ou 4 atomes de carbone ou -CH₂-CF₃ ;
a, b et c représentent chacun indépendamment zéro ou 1 ;
d est zéro, 1 ou 2 ;
e est zéro ou 1 ;
f est zéro, 1, 2, 3 ou 4;
g est zéro ou 1 ;
ou
R3 représente un groupe -(CH₂)ₕ-phényle ou un groupe -O-phényle,
dans lesquels les radicaux phényle sont non substitués ou sont substitués par 1, 2 ou 3 radicaux choisis dans l'ensemble constitué par F, CI, Br, I et par les groupes -Oⱼ-(CH₂)ₖ-CF₃, alcoxy ayant 1, 2, 3 ou 4 atomes de carbone, alkyle ayant 1, 2, 3 ou 4 atomes de carbone et -SO₂CH₃ ;
j est zéro ou 1 ;
k est zéro, 1, 2 ou 3 ;
h est zéro, 1, 2, 3 ou 4 ;
ou
R3 représente un groupe -(CH₂)ₐₐ-hétéroaryle
qui est non substitué ou est substitué par 1, 2 ou 3 radicaux choisis dans l'ensemble constitué par F, Cl, Br, l et par les groupes -O_{bb}-(CH₂)_{cc}-CF₃, alcoxy ayant 1, 2, 3 ou 4 atomes de carbone, alkyle ayant 1, 2, 3 ou 4 atomes de carbone et -SO₂CH₃ ;
bb est zéro ou 1 ;
cc est zéro, 1, 2 ou 3 :
aa est zéro, 1, 2, 3 ou 4 ;
R4 représente un atome d'hydrogène, F, Cl, Br, l, -CN, -SO₂CH₃, un groupe alcoxy ayant 1, 2, 3 ou 4 atomes de carbone, NR11 R12, -O_{dd}-(CH₂)ₑₑ-(CF₂)_{ff}-CF₃, (SO_{gg})ₕₕ-(CH₂)ⱼⱼ,-(CF₂)ₖₖ-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor ;
R11 et R12 représentent, indépendamment l'un de l'autre, un atome d'hydrogène, un groupe alkyle ayant 1, 2, 3 ou 4 atomes de carbone ou -CH₂-CF₃;
dd, ee et ff représentent chacun indépendamment zéro ou 1 ;
gg est zéro, 1 ou 2 ;
hh est zéro ou 1 ;
jj est zéro, 1, 2, 3 ou 4 ;
kk est zéro ou 1 ;
ou
R4 représente un groupe -(CH₂)ₗₗ-phényle ou un groupe -O-phényle,
dans lesquels les radicaux phényle sont non substitués ou sont substitués par 1, 2 ou 3 radicaux choisis dans l'ensemble constitué par F, CI, Br, I et par les groupes -Oₘₘ-(CH₂)ₙₙ-CF₃, alcoxy ayant 1, 2, 3 ou 4 atomes de carbone, alkyle ayant 1, 2, 3 ou 4 atomes de carbone et -SO₂CH₃ ;
mm est zéro ou 1 ;
nn est zéro, 1, 2 ou 3 ;
ll est zéro, 1, 2, 3 ou 4 ;
ou
R4 représente un groupe -(CH₂)ₒₒ-hétéroaryle
qui est non substitué ou est substitué par 1, 2 ou 3 radicaux choisis dans l'ensemble constitué par F, CI, Br, I et par les groupes -Oₚₚ-(CH₂)ᵣᵣ-CF₃, alcoxy ayant 1, 2, 3 ou 4 atomes de carbone, alkyle ayant 1, 2, 3 ou 4 atomes de carbone et -SO₂CH₃ ;
pp est zéro ou 1 ;
rr est zéro, 1, 2 ou 3 :
oo est zéro, 1, 2, 3 ou 4 ;
et sels pharmaceutiquement acceptables de tels composés.

2. Composés de formule I selon la revendication 1, dans lesquels :
R1 représente un atome d'hydrogène, un groupe alkyle ayant 1, 2, 3 ou 4 atomes de carbone, alcoxy ayant 1, 2, 3 ou 4 atomes de carbone, F, CI, Br, I, -CN, NR5R6, -Oₚ-(CH₂)ₙ-(CF₂)ₒ-CF₃ ou -(SOₘ)_{q}-(CH₂)ᵣ-(CF₂)ₛ-CF₃ ;
R5 et R6 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2
n, o, p, q, r et s représentent, indépendamment les uns des autres, zéro ou 1 ;
R2 représente un atome d'hydrogène ou F ;
R3 représente Cl, Br, l, -CN, -SO₂CH₃, un groupe alcoxy ayant 1, 2, 3 ou 4 atomes de carbone, NR9R10,
-Oₐ-(CH₂)_{b}-(CF₂)_{c}-CF₃, -(SO_{d})ₑ-(CH₂)_{f}-(CF₂)_{g}-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor ;
R9 et R10 représentent, indépendamment l'un de l'autre, un atome d'hydrogène, un groupe alkyle ayant 1, 2, 3 ou 4 atomes de carbone ou -CH₂-CF₃ ;
a, b et c représentent chacun indépendamment zéro ou 1 ;
d est zéro, 1 ou 2 ;
e est zéro ou 1 ;
f est zéro, 1, 2, 3 ou 4;
g est zéro ou 1 ;
ou
R3 représente un groupe -(CH₂)ₕ-phényle ou un groupe -O-phényle,
dans lesquels les radicaux phényle sont non substitués ou sont substitués par 1, 2 ou 3 radicaux choisis dans l'ensemble constitué par F, CI, Br, I et par les groupes -Oⱼ-(CH₂)ₖ-CF₃, alcoxy ayant 1, 2, 3 ou 4 atomes de carbone, alkyle ayant 1, 2, 3 ou 4 atomes de carbone et -SO₂CH₃ ;
j est zéro ou 1 ;
k est zéro, 1, 2 ou 3 ;
h est zéro, 1, 2, 3 ou 4 ;
ou
R3 représente un groupe -(CH₂)ₐₐ-hétéroaryle
qui est non substitué ou est substitué par 1, 2 ou 3 radicaux choisis dans l'ensemble constitué par F, Cl, Br, I et par les groupes -O_{bb}-(CH₂)_{cc}-CF₃, alcoxy ayant 1, 2, 3 ou 4 atomes de carbone, alkyle ayant 1, 2, 3 ou 4 atomes de carbone et -SO₂CH₃ ;
bb est zéro ou 1 ;
cc est zéro, 1, 2 ou 3 ;
aa est zéro, 1, 2, 3 ou 4 ;
R4 représente un atome d'hydrogène ou F ;
et sels pharmaceutiquement acceptables de tels composés.

3. Composés de formule I selon la revendication 1 ou 2, dans lesquels :
R1 représente un atome d'hydrogène, un groupe alkyle ayant 1, 2, 3 ou 4 atomes de carbone, alcoxy ayant 1, 2, 3 ou 4 atomes de carbone, F, CI, Br, I, -CN, NR5R6, -O-CH₂-CF₃ ou -(SOₘ)_{q}-(CH₂)ᵣ-CF₃;
R5 et R6 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2
q et r représentent, indépendamment l'un de l'autre, zéro ou 1 ;
R2 représente un atome d'hydrogène ou F ;
R3 représente CI, Br, I, -CN, -SO₂CH₃, un groupe alcoxy ayant 1, 2, 3 ou 4 atomes de carbone, NR9R10, -O-CH₂-CF₃, -(SO_{d})ₑ-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor ;
R9 et R10 représentent, indépendamment l'un de l'autre, un atome d'hydrogène, un groupe alkyle ayant 1, 2, 3 ou 4 atomes de carbone ou -CH₂-CF₃ ;
d est zéro, 1 ou 2 ;
e est zéro ou 1 ;
ou
R3 représente un groupe phényle
qui est non substitué ou est substitué par 1, 2 ou 3 radicaux choisis dans l'ensemble constitué par F, Cl, Br, I et par les groupes -Oⱼ-(CH₂)ₖ-CF₃, alcoxy ayant 1, 2, 3 ou 4 atomes de carbone, alkyle ayant 1, 2, 3 ou 4 atomes de carbone et -SO₂CH₃ ;
j est zéro ou 1 ;
k est zéro, 1, 2 ou 3 ;
ou
R3 représente un groupe hétéroaryle
qui est non substitué ou est substitué par 1, 2 ou 3 radicaux choisis dans l'ensemble constitué par F, Cl, Br, I et par les groupes -O_{bb}-(CH₂)_{cc}-CF₃, alcoxy ayant 1, 2, 3 ou 4 atomes de carbone, alkyle ayant 1, 2, 3 ou 4 atomes de carbone et -SO₂CH₃ ;
bb est zéro ou 1 ;
cc est zéro, 1, 2 ou 3 ;
R4 représente un atome d'hydrogène ou F ;
et sels pharmaceutiquement acceptables de tels composés.

4. Composés de formule I selon une ou plusieurs des revendications 1, 2 ou 3, dans lesquels :
R1 représente un atome d'hydrogène, un groupe alkyle ayant 1, 2, 3 ou 4 atomes de carbone, méthoxy, éthoxy, F, Cl, NR5R6, -O-CH₂-CF₃ ou -(SOₘ)_{q}-(CH₂)ᵣ-CF₃ ;
R5 et R6 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2
q et r représentent, indépendamment l'un de l'autre, zéro ou 1 ;
R2 représente un atome d'hydrogène ou F ;
R3 représente CI, -CN, -SO₂CH₃, un groupe méthoxy, éthoxy, NR9R10, -O-CH₂-CF₃, -(SO_{d})ₑ-CF₃, un groupe alkyle ayant 1, 2, 3, 4, 5 ou 6 atomes de carbone ou cycloalkyle ayant 3, 4, 5, 6 ou 7 atomes de carbone, dans lequel 1, 2, 3 ou 4 atomes d'hydrogène peuvent être remplacés par des atomes de fluor ;
R9 et R10 représentent, indépendamment l'un de l'autre, un groupe méthyle, éthyle ou -CH₂-CF₃ ;
d est zéro, 1 ou 2;
e est zéro ou 1 ;
ou
R3 représente un groupe phényle
qui est non substitué ou est substitué par 1 ou 2 radicaux choisis dans l'ensemble constitué par F, Cl et par les groupes -Oⱼ-(CH₂)ₖ-CF₃, méthoxy, éthoxy, alkyle ayant 1, 2, 3 ou 4 atomes de carbone et -SO₂CH₃ ;
j et k représentent, indépendamment l'un de l'autre, zéro ou 1 ;
ou
R3 représente un groupe hétéroaryle
qui est non substitué ou est substitué par 1 ou 2 radicaux choisis dans l'ensemble constitué par F, CI et par les groupes -O_{bb}-(CH₂)_{cc}-CF₃, méthoxy, éthoxy, alkyle ayant 1, 2, 3 ou 4 atomes de carbone et -SO₂CH₃ ;
bb et cc représentent, indépendamment l'un de l'autre, zéro ou 1 ;
R4 représente un atome d'hydrogène ou F ;
et sels pharmaceutiquement acceptables de tels composés.

5. Composés de formule I selon l'une quelconque des revendications 1 à 4, choisis parmi :
la N-(5-méthanesulfonyl-2-méthyl-4-pentafluorosulfanylbenzoyl)guanidine et ses sels pharmaceutiquement acceptables.

6. Procédé pour la préparation d'un composé de formule l selon une
ou plusieurs des revendications 1, 2, 3, 4 ou 5, ou de ses sels pharmaceutiquement acceptables, **caractérisé en ce qu'**on fait réagir avec de la guanidine un composé de formule II 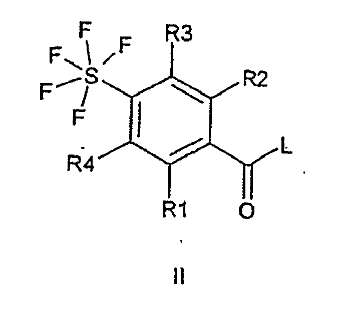 dans laquelle R1 à R4 ont les significations données dans les revendications 1 à 4, et L représente un groupe partant pouvant être remplacé par substitution nucléophile.

7. Composé de formule I ou sels pharmaceutiquement acceptables d'un tel composé, selon une ou plusieurs des revendications 1 à 5, pour utilisation en tant que médicament.

8. Utilisation d'un composé de formule I ou de ses sels pharmaceutiquement acceptables selon une ou plusieurs des revendications 1 à 5, pour la fabrication d'un médicament destiné au traitement ou à la prophylaxie de troubles, de maladies ou de maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulino-dépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure,* de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

9. Utilisation d'un composé de formule I ou de ses sels pharmaceutiquement acceptables selon une ou plusieurs des revendications 1 à 5, en association avec d'autres médicaments ou principes actifs pour la fabrication d'un médicament destiné au traitement ou à la prophylaxie de troubles, de maladies ou de maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulino-dépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure,* de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

10. Utilisation d'un composé de formule I ou de ses sels pharmaceutiquement acceptables selon la revendication 9, en association avec des principes actifs ou des médicaments cardiotoxiques et cytotoxiques, pour la fabrication d'un médicament à propriétés cardiotoxiques et cytotoxiques réduites.

11. Utilisation d'un composé de formule l ou de ses sels pharmaceutiquement acceptables, seul(s) ou en association avec d'autres principes actifs ou médicaments selon la revendication 8 et/ou la revendication 9, pour la fabrication d'un médicament destiné au traitement ou à la prophylaxie de troubles, de maladies ou de maladies secondaires indirectes, chroniques ou aigus, d'organes et de tissus, qui sont provoqués par des incidents ischémiques ou par des incidents de reperfusion.

12. Utilisation d'un composé de formule I ou de ses sels pharmaceutiquement acceptables, seul(s) ou en association avec d'autres principes actifs ou médicaments selon la revendication 8 et/ou la revendication 9, pour la fabrication d'un médicament destiné au traitement de la fibrillation ventriculaire du coeur, mettant la vie en danger.

13. Utilisation d'un composé de formule l ou de ses sels pharmaceutiquement acceptables, seul(s) ou en association avec d'autres principes actifs ou médicaments selon la revendication 8 et/ou la revendication 9, pour la fabrication d'un médicament destiné au traitement ou à la prophylaxie de la dissémination de métastases.

14. Utilisation d'un composé de formule 1 ou de ses sels pharmaceutiquement acceptables, seul(s) ou en association avec d'autres principes actifs ou médicaments selon la revendication 8 et/ou la revendication 9, pour la fabrication d'un médicament destiné au traitement ou à la prophylaxie de maladies fibreuses du coeur, de l'insuffisance cardiaque ou de la *congestive heart failure.*

15. Médicament pour l'utilisation humaine, vétérinaire ou phytosanitaire, contenant une quantité efficace d'au moins un composé de formule I et/ou de ses sels pharmaceutiquement acceptables selon une ou plusieurs des revendications 1 à 5, conjointement avec des véhicules et additifs pharmaceutiquement acceptables.

16. Médicament pour l'utilisation humaine, vétérinaire ou phytosanitaire, contenant une quantité efficace d'au moins un composé de formule I ou de ses sels pharmaceutiquement acceptables selon une ou plusieurs des revendications 1 à 5, conjointement avec des véhicules et additifs pharmaceutiquement acceptables, en association avec d'autres médicaments ou principes actifs pharmacologiques.
